# EUROPEAN PATENT SPECIFICATION

(11) **EP 4 525 760 B1**
(45) Date of publication and mention of the grant of the patent: **11.02.2026**
(21) Application number: 23735370.1
(22) Date of filing: 16.05.2023
(51) Int. Cl.: A61B 18/14, A61B 90/00

(54) **ELECTROSURGICAL TOOLS**
ELEKTROCHIRURGISCHE WERKZEUGE
DISPOSITIFS ÉLECTROCHIRURGICAUX

(30) Priority: 16.05.2022 US 202263342611 P
(43) Date of publication of application: 26.03.2025
(73) Proprietor: Stryker European Operations Limited, Carrigtwohill, Co. Cork T45 HX08 (IE)
(72) Inventor: SHERIDAN, Paul, Enniscorthy Wexford Y21 TK54 (IE); BURKE, Michael, Cobh Cork P24 F857 (IE); FREY, Laura, Constance, Belfast BT9 5GE (IE); MCFARLAND, Scott, Antrim Greenisland BT38 8YB (IE)
(74) Representative: Röthinger, Rainer
(86) International application number: PCT/IB2023/000272
(87) International publication number: WO 2023/223096

(56) References cited:
- CN-U- 209 032 624
- US-A1- 2016 157 920
- US-A1- 2019 000 470
- US-A1- 2019 201 023

## Description

### CROSS-REFERENCE TO RELATED APPLICATION

The present application claims the benefit of priority of U.S. Provisional Application No. 63/342,611, filed May 16, 2022.

### FIELD

The present disclosure generally relates to electrosurgery and, more specifically, to electrosurgical tools.

### BACKGROUND

Electrosurgery involves applying a radio frequency (RF) electric current (also referred to as electrosurgical energy) to biological tissue to cut, coagulate, or modify the biological tissue during an electrosurgical procedure. Specifically, an electrosurgical generator generates and provides the electric current to an active electrode, which applies the electric current (and, thus, electrical power) to the tissue. The electric current passes through the tissue and returns to the generator via a return electrode (also referred to as a "dispersive electrode"). As the electric current passes through the tissue, an impedance of the tissue converts a portion of the electric current into thermal energy (e.g., via the principles of resistive heating), which increases a temperature of the tissue and induces modifications to the tissue (e.g., cutting, coagulating, ablating, and/or sealing the tissue). Document US 2016/0157920 A1 discloses medical devices, systems and methods, and more particularly illuminated electrosurgical instruments such as illuminated energy tips such as electrosurgical, plasma, or laser tips. Document CN 209 032 624 U discloses a multifunctional surgical dissection tool that is easy to operate. This includes a hollow conductive tube, insulated tubes, plastic handles, and blade heads. The blade heads are in a plate-like structure on one side of the end face of the hollow conductive tube and are integrally set with the hollow conductive tube. The plate-like structure of the blade head is bent into an arc-shaped structure along the radial cross-section of the hollow conductive tube, and the width of the blade head is bent toward the center of the hollow conductive tube on both sides. Document US 2019/0201023 A1 discloses surgical systems and grasping instruments that are designed to grasp the tissue of a patient, dissecting instruments configured to manipulate the tissue of a patient, clip appliers configured to clip the tissue of a patient, and suturing instruments configured to suture the tissue of a patient. Document US 2019/0000470 A1 discloses surgical instruments and surgical stapling and cutting instruments and staple cartridges therefor that are designed to staple and cut tissue.

### SUMMARY

According to the invention, an electrosurgical tool includes a handle defining an interior cavity, a power cord configured to couple to and receive electrosurgical energy from an electrosurgical generator, and a shaft extending distally from the interior cavity of the handle. The shaft defines an interior bore. The electrosurgical tool also includes a printed circuit board in the interior bore of the shaft. The printed circuit board is electrically coupled to the power cord. The printed circuit board is configured to conduct the electrosurgical energy from the power cord to an interior surface of the shaft. The shaft is at least one of: (i) rotatable relative to the handle and the printed circuit board or (ii) telescopically movable relative to the handle and the printed circuit board. The electrosurgical tool also includes an electrosurgical electrode extending distally from a distal end of the shaft. The shaft is configured to conduct the electrosurgical energy to the electrosurgical electrode. The invention is as defined in the independent claim. Methods for treatment by surgery or therapy described hereafter do not form part of the claimed apparatus.

In an example useful for understanding the invention, a method of operating an electrosurgical tool includes coupling a power cord of an electrosurgical tool to an electrosurgical generator. The electrosurgical tool includes a handle defining an interior cavity, and a shaft extending distally from the interior cavity of the handle. The shaft defines an interior bore. The electrosurgical tool also includes a printed circuit board in the interior bore of the shaft. The printed circuit board is electrically coupled to the power cord. The printed circuit board is configured to conduct the electrosurgical energy from the power cord to an interior surface of the shaft. The shaft is at least one of: (i) rotatable relative to the handle and the printed circuit board or (ii) telescopically movable relative to the handle and the printed circuit board. The electrosurgical tool also includes an electrosurgical electrode extending distally from a distal end of the shaft.

The method also includes supplying, using the power cord, electrosurgical energy from the electrosurgical generator to the printed circuit board. Additionally, the method includes supplying, by the printed circuit board, the electrosurgical energy from the power cord to an interior surface of the shaft. The method further includes conducting the electrosurgical energy from the shaft to the electrosurgical electrode.

In another example useful for understanding the invention, a method of making an electrosurgical tool includes forming a housing including a handle defining an interior cavity and a shaft extending distally from the interior cavity of the handle. The shaft defines an interior bore. The method also includes positioning a printed circuit board in the interior bore of the shaft, and electrically coupling the printed circuit board to a power cord. The power cord is configured to couple to and receive electrosurgical energy from an electrosurgical generator. The shaft is at least one of: (i) rotatable relative to the handle and the printed circuit board or (ii) telescopically movable relative to the handle and the printed circuit board. The method also includes electrically coupling the printed circuit board to an interior surface of the shaft, and electrically coupling an electrosurgical electrode to a distal end of the shaft. The shaft is configured to conduct the electrosurgical energy to the electrosurgical electrode.

### BRIEF DESCRIPTION OF THE FIGURES

The novel features believed characteristic of the illustrative examples are set forth in the appended claims. The illustrative examples, however, as well as a preferred mode of use, further objectives and descriptions thereof, will best be understood by reference to the following detailed description of an illustrative example of the present disclosure when read in conjunction with the accompanying drawings, wherein:
Figure 1 depicts a simplified block diagram of an electrosurgical system, according to an example.
Figure 2 illustrates a partial cross-sectional view of an electrosurgical tool, according to an example.
Figure 3 depicts a perspective view of an electrosurgical tool according to an example.
Figure 4 depicts a cross-sectional view of the electrosurgical tool shown in Figure 3, according to an example.
Figure 5 depicts a partial cross-sectional view of a shaft of the electrosurgical tool shown in Figure 3, according to an example.
Figure 6 depicts a partial cross-sectional view of a shaft of the electrosurgical tool shown in Figure 3, according to another example.
Figure 7A depicts a first side view of a printed circuit board, according to an example.
Figure 7B depicts a second side view of the printed circuit board shown in Figure 7A, according to an example.
Figure 8 depicts a printed circuit board, according to another example.
Figure 9 depicts an enlarged view of the cross-section of a distal end of a shaft and an electrosurgical electrode, according to an example.
Figure 10 depicts a perspective view of a smoke evacuation channel and a shaft of an electrosurgical tool, according to an example.
Figure 11 depicts a side view of the smoke evacuation channel and the shaft of an electrosurgical tool, according to an example.
Figure 12 depicts a partially exploded view of an assembly of a printed circuit board, a light source, an optical structure, a heat sink, and a smoke evacuation channel of an electrosurgical, according to an example.
Figure 13 depicts an assembly of an end of a printed circuit board, an optical structure, and an electrosurgical electrode, according to an example.
Figure 14 depicts a side view of the electrosurgical tool with a first portion of the handle omitted for illustration purposes, according to an example.
Figure 15 depicts a first perspective view of the electrosurgical tool shown in Figure 14 with a second portion of the handle omitted for illustration purposes, according to an example.
Figure 16 depicts a second perspective view of the electrosurgical tool shown in Figure 14 with the second portion of the handle omitted for illustration purposes, according to an example.
Figure 17 depicts the electrosurgical tool shown in Figure 14 with both the first portion and the second portion of the handle omitted for illustration purposes, according to an example.
Figure 18 depicts an exploded view of a stop assembly of the electrosurgical tool shown in Figure 14, according to an example.
Figure 19 depicts a shaft stop of the stop assembly of the electrosurgical tool shown in Figure 14, according to an example.
Figure 20 depicts a partial cross-sectional view of a coupling between the shaft stop and the shaft for the electrosurgical tool shown in Figure 14, according to an example.
Figure 21 depicts a rotation nut of the electrosurgical tool shown in Figure 14, according to an example.
Figure 22 depicts a flowchart for a process of operating an electrosurgical tool, according to an example.
Figure 23 depicts a flowchart for a process of operating an electrosurgical tool that can be used with at least the process shown in Figure 22, according to an example.
Figure 24 depicts a flowchart for a process of operating an electrosurgical tool that can be used with at least the process shown in Figure 22, according to an example.
Figure 25 depicts a flowchart for a process of operating an electrosurgical tool that can be used with at least the process shown in Figure 22, according to an example.
Figure 26 depicts a flowchart for a process of operating an electrosurgical tool that can be used with at least the process shown in Figure 22, according to an example.
Figure 27 depicts a flowchart for a process of operating an electrosurgical tool that can be used with at least the process shown in Figure 22, according to an example.
Figure 28 depicts a flowchart for a process of operating an electrosurgical tool that can be used with at least the process shown in Figure 22, according to an example.
Figure 29 depicts a flowchart for a process of operating an electrosurgical tool that can be used with at least the process shown in Figure 22, according to an example.
Figure 30 depicts a flowchart for a process of operating an electrosurgical tool that can be used with at least the process shown in Figure 22, according to an example.
Figure 31 depicts a flowchart for a process of operating an electrosurgical tool that can be used with at least the process shown in Figure 22, according to an example.
Figure 32 depicts a flowchart for a process of operating an electrosurgical tool that can be used with at least the process shown in Figure 22, according to an example.
Figure 33 depicts a flowchart for a process of operating an electrosurgical tool that can be used with at least the process shown in Figure 22, according to an example.
Figure 34 depicts a flowchart for a process of making an electrosurgical tool, according to an example.
Figure 35 depicts a flowchart for a process of making an electrosurgical tool that can be used with at least the process shown in Figure 34, according to an example.
Figure 36 depicts a flowchart for a process of making an electrosurgical tool that can be used with at least the process shown in Figure 34, according to an example.
Figure 37 depicts a flowchart for a process of making an electrosurgical tool that can be used with at least the process shown in Figure 34, according to an example.
Figure 38 depicts a flowchart for a process of making an electrosurgical tool that can be used with at least the process shown in Figure 34, according to an example.
Figure 39 depicts a flowchart for a process of making an electrosurgical tool that can be used with at least the process shown in Figure 34, according to an example.
Figure 40 depicts a flowchart for a process of making an electrosurgical tool that can be used with at least the process shown in Figure 34, according to an example.
Figure 41 depicts a flowchart for a process of making an electrosurgical tool that can be used with at least the process shown in Figure 34, according to an example.
Figure 42 depicts a flowchart for a process of making an electrosurgical tool that can be used with at least the process shown in Figure 34, according to an example.
Figure 43 depicts a flowchart for a process of making an electrosurgical tool that can be used with at least the process shown in Figure 34, according to an example.
Figure 44 depicts a flowchart for a process of making an electrosurgical tool that can be used with at least the process shown in Figure 34, according to an example.
Figure 45 depicts a flowchart for a process of making an electrosurgical tool that can be used with at least the process shown in Figure 34, according to an example.
Figure 46 depicts a cross-sectional view of an electrosurgical tool through a handle, a shaft stop, and a rotational nut, according to another example.

### DETAILED DESCRIPTION

Disclosed examples will now be described more fully hereinafter with reference to the accompanying drawings, in which some, but not all of the disclosed examples are shown. Indeed, several different examples may be described and should not be construed as limited to the examples set forth herein. Rather, these examples are described so that this disclosure will be thorough and complete and will fully convey the scope of the disclosure to those skilled in the art.

By the term "approximately" or "substantially" with reference to amounts or measurement values described herein, it is meant that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

As noted above, an electrosurgical tool can use electrical energy supplied by an electrosurgical generator to apply electrosurgical energy from an electrosurgical electrode to a tissue. As such, the electrosurgical tool generally includes a housing in which one or more conductors are disposed for supplying the electrosurgical energy to the electrosurgical electrode. Some electrosurgical tools include a shaft that is axially adjustable relative to the housing (e.g., the shaft may be telescopically movable relative to the housing). This can facilitate adjusting a length of the electrosurgical tool to treat differently sized and/or shaped target tissues.

Additionally, some electrosurgical tools provide for rotation of the electrosurgical electrode relative to the housing. This can facilitate adjusting an angle of the electrosurgical electrode relative to one or more user input device(s) of the electrosurgical tool. In this arrangement, a user can comfortably grip the housing in a position in which their fingers can comfortably operate the user input device(s) while the electrosurgical electrode is set at a rotational position selected from among a plurality of rotational positions relative to the housing based on, for example, a location, a size, and/or a shape of a surgical site in which the user is operating.

However, providing for axial movement and/or rotation of the electrosurgical electrode relative to the housing can increase design complexity and a cost of manufacture. For instance, it can be challenging to maintain the electrical connection between electrical components in the housing and the electrosurgical electrode when the electrosurgical electrode rotates relative to the housing and/or axially moves relative to the housing. This problem may be further compounded when the electrosurgical tool includes other features distal of the housing (e.g., a light source, one or more optical components, and/or smoke evacuation features).

The present application provides for electrosurgical tools that can address at least some of the challenges described above. For instance, in one example, an electrosurgical tool can include a printed circuit board in an interior bore of a shaft, which extends distally from an interior cavity of a housing. The printed circuit board can conduct electrosurgical energy from a power cord to an interior surface of the shaft. The shaft can be movable relative to the housing and the printed circuit board. An electrosurgical electrode can extend distally from a distal end of the shaft, and receive the electrosurgical energy from the shaft. In this arrangement, the printed circuit board and the shaft can maintain an electrical connection between the electrosurgical electrode and an source of the electrosurgical energy (e.g., an electrosurgical generator) in a plurality of rotational orientations and/or axial positions of the electrosurgical electrode relative to the housing.

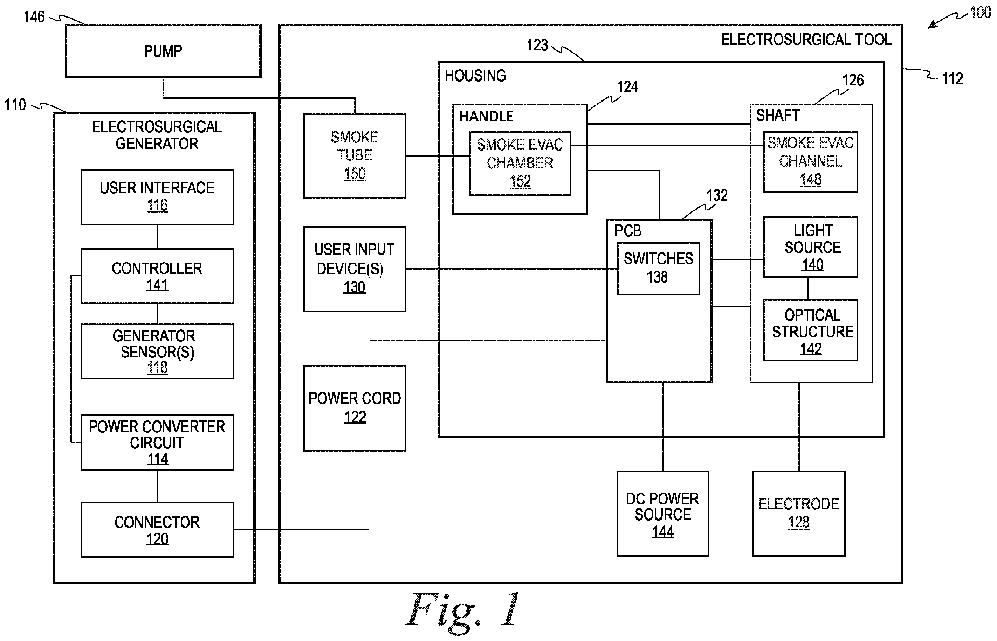

Referring now to Figure 1, an electrosurgical system 100 is shown according to an example. As shown in Figure 1, the electrosurgical system 100 includes an electrosurgical generator 110 and an electrosurgical tool 112. In general, the electrosurgical generator 110 can generate electrosurgical energy that is suitable for performing electrosurgery on a patient. For instance, the electrosurgical generator 110 can include a power converter circuit 114 that can convert a grid power to electrosurgical energy such as, for example, a radio frequency (RF) output power. As an example, the power converter circuit 114 can include one or more electrical components (e.g., one or more transformers) that can control a voltage, a current, and/or a frequency of the electrosurgical energy.

Within examples, the electrosurgical generator 110 can include a user interface 116 that can receive one or more inputs from a user and/or provide one or more outputs to the user. As examples, the user interface 116 can include one or more buttons, one or more switches, one or more dials, one or more keypads, one or more touchscreens, one or more display screens, one or more indicator lights, one or more speakers, and/or one or more haptic output devices.

In an example, the user interface 116 can be operable to select a mode of operation from among a plurality of modes of operation for the electrosurgical generator 110. As examples, the modes of operation can include a cutting mode, a coagulating mode, an ablating mode, and/or a sealing mode. Combinations of these waveforms can also be formed to create blended modes. In one implementation, the modes of operation can correspond to respective waveforms for the electrosurgical energy. As such, in this implementation, the electrosurgical generator 110 can generate the electrosurgical energy with a waveform selected from a plurality of waveforms based, at least in part, on the mode of operation selected using the user interface 116.

The electrosurgical generator 110 can also include one or more generator sensors 118 that can sense one or more conditions related to the electrosurgical energy and/or the target tissue. As examples, the generator sensor(s) 118 can include one or more current sensors, one or more voltage sensors, one or more temperature sensors, and/or one or more bioimpedance sensors. Within examples, the electrosurgical generator 110 can additionally or alternatively generate the electrosurgical energy with an amount of electrosurgical energy (e.g., an electrical power) and/or a waveform selected from among the plurality of waveforms based on one or more parameters related to the condition(s) sensed by the generator sensor(s) 118.

In one example, the electrosurgical energy can have a frequency that is greater than approximately 100 kilohertz (kHz) to reduce (or avoid) stimulating a muscle and/or a nerve near the target tissue. In another example, the electrosurgical energy can have a frequency that is between approximately 300 kHz and approximately 500 kHz.

In Figure 1, the electrosurgical generator 110 also includes a connector 120 that can facilitate coupling the electrosurgical generator 110 to the electrosurgical tool 112. For example, the electrosurgical tool 112 can include a power cord 122 having a plug, which can be coupled to a socket of the connector 120 of the electrosurgical generator 110. In this arrangement, the electrosurgical generator 110 can supply the electrosurgical energy to the electrosurgical tool 112 via the coupling between the connector 120 of the electrosurgical generator 110 and the power cord 122 of the electrosurgical tool 112.

The electrosurgical generator 110 can further include a controller 141 that can control operation of the electrosurgical generator 110. Within examples, the controller 141 can be implemented using hardware, software, and/or firmware. For instance, the controller 141 can include one or more processors and a non-transitory computer readable medium (e.g., volatile and/or non-volatile memory) that stores machine language instructions or other executable instructions. The instructions, when executed by the one or more processors, cause the electrosurgical generator 110 to carry out the various operations described herein. The controller 141, thus, can receive data and store the data in the memory as well. As shown in Figure 1, the controller 141 can be communicatively coupled with the power converter circuit 114, the user interface 116, the generator sensor(s) 118, and/or the connector 120.

As shown in Figure 1, the electrosurgical tool 112 can include a housing 123. The housing 123 can be an elongated structure in and/or on which components of the electrosurgical tool 112 can be disposed. In some examples, the housing 123 can be an integral, monolithic structure. In other examples the housing 123 can include a plurality of structures that are coupled to each other.

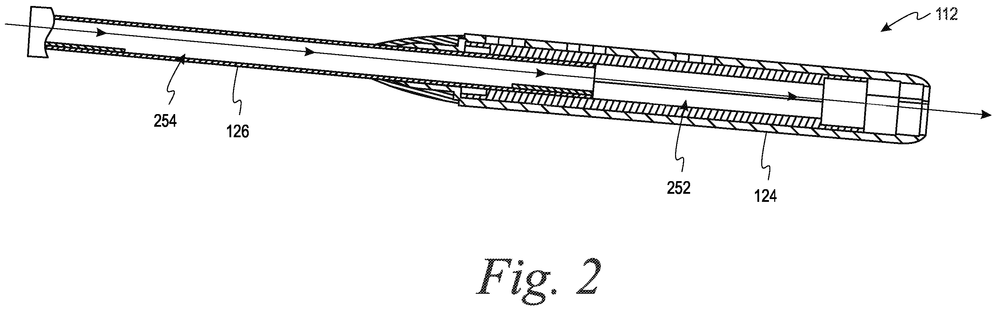

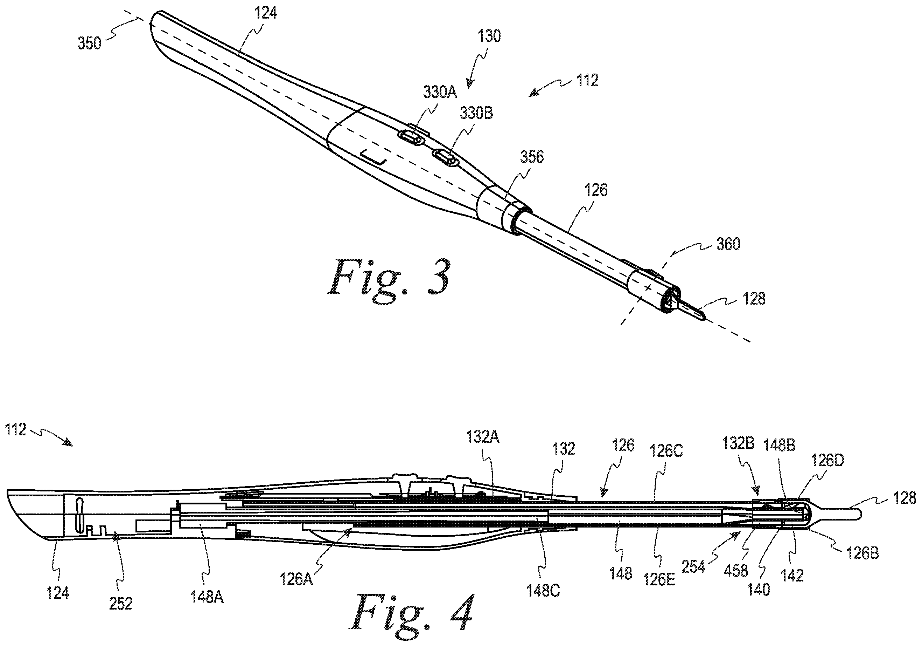

In Figure 1, the housing 123 includes a handle 124 that defines an interior cavity 252 (e.g., shown in Figures 2 and 4), a shaft 126 extending in a distal direction from the handle 124 and defining an interior bore 254 (e.g., shown in Figures 2 and 4), and a monopolar electrosurgical electrode 128 extending in the distal direction from the shaft 126. In general, the handle 124 can be configured to facilitate a user gripping and manipulating the electrosurgical tool 112 while performing electrosurgery. For example, the handle 124 can have a shape and/or a size that can facilitate a user performing electrosurgery by manipulating the electrosurgical tool 112 using a single hand. In one implementation, the handle 124 can have a shape and/or a size that facilitates the user holding the electrosurgical tool 112 in a writing utensil gripping manner (e.g., the electrosurgical tool 112 can be an electrosurgical pencil).

Additionally, for example, the handle 124 and/or the shaft 126 can include one or more materials that are electrical insulators (e.g., a plastic material). This can facilitate insulating the user from the electrosurgical energy flowing through the electrosurgical tool 112 while performing the electrosurgery. As described in further detail below, at least a portion of the shaft 126 is formed from an electrically conductive material such that the shaft 126 can conduct the electrosurgical energy to the monopolar electrosurgical electrode 128.

In some implementations, the shaft 126 can be coupled to the handle 124 in a fixed and non-moveable manner. This may simplify manufacturing and reduce a cost of manufacture by, for instance, simplifying electrical connections that may otherwise need to account for movement of the shaft 126 and the handle 124 relative to each other (e.g., by omitting slip ring electrical contacts and/or sliding electrical contacts). In one example, the handle 124 and the shaft 126 can be formed as a single, monolithic structure such that the shaft 126 and the handle 124 are fixed and non-moveable relative to each other. In another example, the handle 124 and the shaft 126 can be fixedly coupled to each other by a welding coupling, an adhesive coupling, and/or another coupling that prevents movement between the handle 124 and the shaft 126.

In other implementations, the shaft 126 can be moveable relative to the handle 124 along a longitudinal axis of the electrosurgical tool 112. For example, the shaft 126 can be telescopically moveable in the interior cavity 252 defined by the handle 124 to extend the shaft 126 in the distal direction and retract the shaft 126 in a proximal direction relative to the handle 124 (e.g., movable along a longitudinal axis of the electrosurgical tool 112). The monopolar electrosurgical electrode 128 is coupled to the shaft 126 and, thus, the monopolar electrosurgical electrode 128 can move together with the shaft 126 in an axial direction along the longitudinal axis relative to the handle 124. This can provide for adjusting a length of the electrosurgical tool 112, which can facilitate performing electrosurgery at a plurality of different depths within tissue (e.g., due to different anatomical shapes and/or sizes of patients) and/or at a plurality of different angles.

In some implementations, the monopolar electrosurgical electrode 128 can additionally or alternatively be rotatable about an axis of rotation that is parallel to the longitudinal axis of the electrosurgical tool 112. In some examples, the monopolar electrosurgical electrode 128 can be rotatable relative to the handle 124 and the shaft 126. In other examples, the monopolar electrosurgical electrode 128 can be rotationally fixed relative to the shaft 126 such that the shaft 126 and the monopolar electrosurgical electrode 128 are rotatable together relative to the handle 124. Rotating the monopolar electrosurgical electrode 128 relative to the handle 124 can facilitate adjusting an angle of the monopolar electrosurgical electrode 128 relative to one or more user input device(s) 130 of the electrosurgical tool 112. In this arrangement, a user can comfortably grip the handle 124 in a position in which their fingers can comfortably operate the user input device(s) 130 while the monopolar electrosurgical electrode 128 is set at a rotational position selected from among a plurality of rotational positions relative to the handle 124 based on, for example, a location, a size, and/or a shape of a surgical site in which the user is operating.

In one implementation, the monopolar electrosurgical electrode 128 can be rotatable by more than 360 degrees relative to the handle 124. This can improve an ease of use by allowing an operator to freely rotate the monopolar electrosurgical electrode 128 without limitation. However, in other implementations, the monopolar electrosurgical electrode 128 can be rotatable by less than or equal to 360 degrees (e.g., rotatable by 180 degrees or rotatable by 360 degrees). This may still allow an operator to achieve a desired rotational arrangement, but with the possibility that the operator may rotate in first direction, reach a stop limiting further rotation, and then rotate back in a second direction to achieve the desired rotational arrangement.

Although it can be beneficial to provide for rotation of the monopolar electrosurgical electrode 128 relative to the handle 124 and/or the shaft 126, the monopolar electrosurgical electrode 128 can be rotationally fixed relative to the handle 124 and the shaft 126 in some implementations. This may, for example, help to simplify manufacturing and reduce a cost of manufacture by, for instance, simplifying electrical connections that may otherwise need to account for movement of the shaft 126 and the handle 124 relative to each other (e.g., by omitting slip ring electrical contacts and/or sliding electrical contacts).

The user input device(s) 130 can select between the modes of operation of the electrosurgical tool 112 and/or the electrosurgical generator 110. For instance, in one implementation, the user input device(s) 130 can be configured to select between a cutting mode of operation and a coagulation mode of operation. Responsive to actuation of the user input device(s) 130 of the electrosurgical tool 112, the electrosurgical tool 112 can (i) receive the electrosurgical energy with a level of power and/or a waveform corresponding to the mode of operation selected via the user input device(s) 130 and (ii) supply the electrosurgical energy to the monopolar electrosurgical electrode 128.

In Figure 1, the electrosurgical tool 112 includes a plurality of electrical components that facilitate supplying the electrosurgical energy, which the electrosurgical tool 112 receives from the electrosurgical generator 110, to the monopolar electrosurgical electrode 128. For example, at least a portion of the shaft 126 is formed of an electrically conductive material such that the shaft 126 is electrically coupled to the monopolar electrosurgical electrode 128. In one implementation, the shaft 126 can be entirely formed from the electrically conductive material. For instance, the shaft 126 can be a tubular structure formed entirely from a metal. In another implementation, the shaft 126 can include an electrically conductive portion and an insulator portion so long as the electrically conductive portion is configured to conduct the electrosurgical energy to the monopolar electrosurgical electrode 128.

Additionally, the electrosurgical tool 112 includes a printed circuit board 132 (e.g., a flexible printed circuit board) in an interior bore 254 of the shaft 126 and/or the interior cavity 252 of the handle 124. The printed circuit board 132 can be electrically coupled with the power cord 122 and the shaft 126, which is electrically coupled with the monopolar electrosurgical electrode 128. In this arrangement, the printed circuit board 132 and the shaft 126 provide a circuit for conducting the electrosurgical energy from the power cord 122 to the monopolar electrosurgical electrode 128.

As described in further detail below, the electrosurgical tool 112 can include one or more components that can help to maintain the electrical coupling between the printed circuit board 132 and an interior surface of the shaft 126 (and, thus, the monopolar electrosurgical electrode 128) in a plurality of axial positions of the shaft 126 relative to the handle 124 and/or a plurality of rotational orientations of the shaft 126 relative to the handle 124 (e.g., in all axial positions as the shaft 126 telescopically moves relative to the handle 124 and/or in all rotational orientations as the shaft 126 rotates relative to the handle 124).

Within examples, the user input device(s) 130 can include one or more buttons on an exterior surface of the handle 124. Each button of the user input device(s) 130 can be operable to actuate a respective one of a plurality of switches 138 of the printed circuit board 132. In general, the switches 138 and/or the printed circuit board 132 are operable to control a supply of the electrosurgical energy from the electrosurgical generator 110 to the monopolar electrosurgical electrode 128. For instance, in one implementation, when each button is operated (e.g., depressed), the respective switch 138 associated with the button can be actuated to cause the printed circuit board 132 to transmit a signal to the electrosurgical generator 110 and cause the electrosurgical generator 110 to responsively supply the electrosurgical energy with a level of power and/or a waveform corresponding to a mode of operation associated with the button. In another implementation, operating the button and thereby actuating the respective switch 138 associated with the button can close the switch 138 to complete a circuit to the electrosurgical generator 110 to cause the electrosurgical generator 110 to responsively supply the electrosurgical energy with a level of power and/or a waveform corresponding to a mode of operation associated with the button. In both example implementations, the electrosurgical energy supplied by the electrosurgical generator 110 can be supplied from the electrosurgical generator 110 to the monopolar electrosurgical electrode 128 by the power cord 122, the printed circuit board 132, and the shaft 126.

Although the electrosurgical tool 112 includes the user input device(s) 130 in Figure 1, the user input device(s) 130 can be separate from the electrosurgical tool 112 in another example. For instance, the user input device(s) 130 can additionally or alternatively include one or more foot pedals that are actuatable to control operation of the electrosurgical tool 112 as described above. The foot pedal(s) can be communicatively coupled to the electrosurgical generator 110 to provide a signal responsive to actuation of the foot pedal(s).

In some examples, the electrosurgical tool 112 can additionally include one or more light sources 140 that are configured to emit light. In some implementations, the light source(s) 140 can be located at a distal end of the housing 123 and/or a distal end of the shaft 126 to directly provide light in a distal direction and illuminate a surgical distal of the monopolar electrosurgical electrode 128.

In other implementations, as shown in Figure **1****,** the light source(s) 140 can be optically coupled to an optical structure 142, which is configured to receive the light emitted by the light source(s) 140 and transmit the light in a distal direction toward a surgical site to illuminate the surgical site while performing electrosurgery using the monopolar electrosurgical electrode 128. Although arranging the light source(s) 140 to directly illuminate a surgical field can help, for instance, to reduce a cost of manufacture, transmitting the light using the optical structure 142 can help to improve a quality of light transmitted from the electrosurgical tool 112 (e.g., by providing light with improved uniformity and/or reduced heat generation).

As examples, in implementations that include the optical structure 142, the optical structure 142 can include at least one optical structure selected from among a group consisting of an optical lens, a non-fiber optic optical waveguide, and an optical fiber. When the optical structure 142 includes the optical lens (e.g., a parabolic reflector lens, an aspheric lens, and/or a Fresnel lens), the optical structure 142 can help to direct the light emitted by the light source 140 in the distal direction and thereby improve a quality of the light illuminating the surgical site. The optical structure 142 can additionally or alternatively include the non-fiber optic optical waveguide and/or the optical fiber to transmit the light over relatively large distances in the shaft 126. For instance, the optical waveguide can transmit the light in the distal direction via total internal reflection. In such implementations, the optical waveguide can include a cladding and/or an air gap on an exterior surface of the optical waveguide to help facilitate total internal reflection. In some implementations, the non-fiber optic optical waveguide can be formed as a single, monolithic structure.

In some examples, the optical structure 142 can additionally or alternatively include other light shaping optical elements such as, for instance, a plurality of facets, one or more prisms, and/or one or more optical gratings. Although the optical structure 142 can help to improve a quality of the light directed to the surgical site, the electrosurgical tool 112 can omit the optical structure 142 and instead emit the light from the light source 140 directly to the surgical field without transmitting the light through the optical structure 142 in other examples.

In Figure 1, the light source 140 can be coupled to the shaft 126. As such, the light source 140 can also move telescopically with the shaft 126 relative to the handle 124. However, in other examples, the light source 140 can be in the interior cavity 252 of the handle 124 and/or coupled to an exterior surface of the handle 124. As examples, the light source 140 can include one or more light emitting diodes (LEDs), organic light emitting diodes (OLEDs), optical fibers, non-fiber optic waveguides, and/or lenses. Additionally, for example, the light source 140 can include a light-emitting diode printed circuit board (LED PCB) having one or more light sources (e.g., LEDs).

The optical structure 142 can be at a distal end of the shaft 126. In some examples, the optical structure 142 can circumferentially surround the monopolar electrosurgical electrode 128 to emit the light distally around all sides of the monopolar electrosurgical electrode 128. This can help to mitigate shadows and provide greater uniformity of illumination in all rotational alignments of the shaft 126 relative to the housing 123 and/or the electrosurgical tool 112 relative to the target tissue. However, in other examples, the optical structure 142 can extend partially but not fully around the monopolar electrosurgical electrode 128.

In implementations that include the light source 140, the user input device(s) 130, the printed circuit board 132, the switches 138, the housing conductor 134, and/or the shaft conductor 136 can additionally supply an electrical power from a direct current (DC) power source 144 to the light source 140. In one example, the DC power source 144 can include a battery disposed in the handle 124, the plug of the power cord 122, and/or a battery receptacle located along the power cord 122 between the handle 124 and the plug. Although the electrosurgical tool 112 includes the DC power source 144 in Figure 1, the DC power source 144 can be separate and distinct from the electrosurgical tool 112 in other examples. For instance, in another example, the electrosurgical generator 110 can include the DC power source 144.

Additionally, in implementations that include the light source 140, the user input device(s) 130 can be operable to cause the light source 140 to emit the light. In one example, the user input device(s) 130 can include a button that independently controls the light source 140 separate from the button(s) that control the electrosurgical operational modes of the electrosurgical tool 112. In another example, the user input device(s) 130 and the printed circuit board 132 can be configured such that operation of the button(s) that control the electrosurgical operational mode simultaneously control operation of the light source 140 (e.g., the light source 140 can be automatically actuated to emit light when a button is operated to apply the electrosurgical energy at the monopolar electrosurgical electrode 128).

As shown in Figure 1, responsive to operation of the user input device(s) 130 to actuate the light source 140, the DC power source 144 can supply the electrical power (e.g., a DC voltage) to the light source 140 via the printed circuit board 132, the housing conductor 134, and/or the shaft conductor 136. In this implementation, one or more of the conductive elements of the housing conductor 134 can be configured to supply the electrical power from the DC power source 144 to the light source 140 and/or return the electrical power from the light source 140 to the DC power source 144. Accordingly, the housing conductor 134 can additionally or alternatively assist in providing electrical communication between the DC power source 144 and the light source 140 as the shaft 126 and the light source 140 telescopically move relative to the handle 124.

Although the user input device(s) 130 on the handle 124 can be operated to control the operation of the light source 140 in the examples described above, the light source 140 can be additionally or alternatively operated by one or more user input device(s) on the electrosurgical generator 110 (e.g., via the user interface 116) and/or on the plug of the power cord 122.

Within examples, the electrosurgical tool 112 can additionally or alternatively include features that provide for evacuating surgical smoke from a target tissue to a location external to the surgical site. Surgical smoke is a by-product of various surgical procedures. For example, during surgical procedures, surgical smoke may be generated as a by-product of electrosurgical units (ESU), lasers, electrocautery devices, ultrasonic devices, and/or other powered surgical instruments (e.g., bones saws and/or drills). In some instances, the surgical smoke may contain toxic gases and/or biological products that result from a destruction of tissue. Additionally, the surgical smoke may contain an unpleasant odor. For these and other reasons, many guidelines indicate that exposure of surgical personnel to surgical smoke should be reduced or minimized.

To reduce (or minimize) exposure to surgical smoke, a smoke evacuation system may be used during the surgical procedure. In general, the smoke evacuation system may include a suction pump 146 that can generate sufficient suction and/or vacuum pressure to draw the surgical smoke away from the surgical site. In some implementations, the smoke evacuation system may be coupled to an exhaust system (e.g., an in-wall exhaust system) that exhausts the surgical smoke out of an operating room. In other implementations, the smoke evacuation system may filter air containing the surgical smoke and return the air to the operating room. Within examples, the suction pump 146 and the electrosurgical generator 110 can be provided as separate devices or integrated in a single device (e.g., in a common housing).

As shown in Figure 1, the shaft 126 can include a smoke evacuation channel 148 in the interior bore 254 of the shaft 126. The smoke evacuation channel 148 can also include a smoke inlet that can extend circumferentially around a center axis of a distal portion of the monopolar electrosurgical electrode 128. In this arrangement, the smoke inlet of the smoke evacuation channel can help to receive surgical smoke into the smoke evacuation channel 148 in all rotational alignments of the monopolar electrosurgical electrode 128 relative to the handle 124 and/or the electrosurgical tool 112 relative to the target tissue. However, in another example, the smoke evacuation channel 148 can include one or more smoke inlets that do not extend circumferentially around the monopolar electrosurgical electrode 128.

In an example, the smoke evacuation channel 148 can include an outer tube that is separated from the optical structure 142 by an air gap. For instance, the shaft 126 can include a plurality of standoffs that extend between the optical structure 142 and the outer tube of the smoke evacuation channel 148 to provide the air gap between the outer tube and the optical structure 142. In one implementation, the optical structure 142 can include the standoffs such that the optical structure 142 and the standoffs are formed as a single, monolithic structure. In another implementation, the standoffs can be formed as a single, monolithic structure with the outer tube of the smoke evacuation channel 148. In another implementation, the standoffs can be separate from the outer tube of the smoke evacuation channel 148 and the optical structure 142.

In an example, the smoke evacuation channel 148 of the shaft 126 defines a first portion of a smoke flow path, and the interior cavity 252 of the handle 124 defines a second portion of a smoke flow path. Figure 2 illustrates a partial cross-sectional view of the electrosurgical tool 112 according to an implementation of this example. In this arrangement, the surgical smoke can be received from the surgical site into the smoke evacuation channel 148 provided by the interior bore 254 of the shaft 126, and flow proximally along the smoke evacuation channel 148 to the interior cavity 252 of the handle 124. In the interior cavity 252 of the handle 124, the smoke can further flow to a smoke tube 150 that is coupled to a proximal end of the handle 124 and configured to convey smoke from the handle 124 to the suction pump 146.

As noted above, the monopolar electrosurgical electrode 128 can apply the electrosurgical energy to a target tissue to perform an electrosurgical operation (e.g., cutting, coagulating, ablating, and/or sealing the target tissue). Within examples, the monopolar electrosurgical electrode 128 includes an electrosurgical substrate formed from an electrically conductive material. As an example, the electrically conductive material can be stainless steel.

As described in further detail below, the electrosurgical substrate can extend in an axial direction from a proximal end of the monopolar electrosurgical electrode 128 to a distal end of the monopolar electrosurgical electrode 128. The proximal end of the monopolar electrosurgical electrode 128 can receive electrosurgical energy from the electrosurgical tool 112 (e.g., via the housing conductor 134 and the shaft conductor 136 as described above), and a distal working portion of the monopolar electrosurgical electrode 128 can apply the electrosurgical energy to the target tissue. In one implementation, the electrosurgical substrate can include a shank portion that extends from the proximal end of monopolar electrosurgical electrode 128 to the distal working portion of the monopolar electrosurgical electrode 128. The distal working portion can be configured for at least one of cutting or coagulation of tissue by the electrosurgical energy received from the electrosurgical tool 112.

In some examples, the distal working portion can define an electrosurgical blade. For instance, the electrosurgical blade can include (i) a first lateral surface, (ii) a second lateral surface opposite the first lateral surface, (iii) a first major surface extending between the first lateral surface and the second lateral surface on a first side of the electrosurgical blade, and (iv) a second major surface extending between the first lateral surface and the second lateral surface on a second side of the electrosurgical blade that is opposite the first side. The first lateral surface and the second lateral surface have surface areas that are relatively small compared to surface areas of the first major surface and the second major surface such that a thickness (e.g., a dimension between the first major surface and the second major surface) of the electrosurgical blade is relatively small as compared to a length (e.g., a dimension extending between the proximal end and the distal end of the monopolar electrosurgical electrode 128) and a width (e.g., a dimension between the first latera surface and the second lateral surface).

Referring now to Figures 3-13, an implementation of the electrosurgical tool 112 is shown according to an example. Figure 3 depicts a perspective view of the electrosurgical tool 112 according to the example. Figure 4 depicts a cross-sectional view of the electrosurgical tool 112 taken through a longitudinal axis 350 of the electrosurgical tool according to the example.

As shown in Figures 3-4, the electrosurgical tool 112 includes the handle 124 defining the interior cavity 252, and the shaft 126 extending distally from the interior cavity 252 of the handle 124. As described above, the shaft 126 defines the interior bore 254. The shaft 126 has a longitudinal axis 350 extending between a proximal end 126A of the shaft 126 and a distal end 126B of the shaft 126. Additionally, the monopolar electrosurgical electrode 128 extends distally from a distal end 126B of the shaft 126. In this implementation, the monopolar electrosurgical electrode 128 is directly coupled or integrally formed with the shaft 126. However, in another implementation, the monopolar electrosurgical electrode 128 can be indirectly coupled to the shaft 126 by an intermediate conductor such that the monopolar electrosurgical electrode 128 receives the electrosurgical energy conducted by the printed circuit board 132, the shaft 126, and the intermediate conductor.

In the example shown in Figures 3-13, the shaft 126 is telescopically movable in the interior cavity 252 of the handle 124 to adjust a distance of a distalmost tip of the monopolar electrosurgical electrode 128 relative to the handle 124. As noted above, telescopically moving the shaft 126 relative to the handle 124 can facilitate adjusting a length of the electrosurgical tool 112 to treat differently sized and/or shaped target tissues. However, as described above, the shaft 126 can be axially fixed to the handle 124 (e.g., fixedly coupled to the handle 124) such that the shaft 126 is not axially moveable relative to the handle 124 in other examples.

Additionally, in this example, the shaft 126 is rotatable relative to the handle 124. As the monopolar electrosurgical electrode 128 is fixedly coupled to the shaft 126 in this example, rotating the shaft 126 causes corresponding rotation of the monopolar electrosurgical electrode 128 relative to the handle 124. As described above, rotating the monopolar electrosurgical electrode 128 relative to the handle 124 can facilitate adjusting an angle of the monopolar electrosurgical electrode 128 relative to one or more user input device(s) 130 of the electrosurgical tool 112. However, as described above, the shaft 126 can be rotationally fixed relative to the handle 124 such that the shaft 126 is not rotatable relative to the handle 124 in other examples.

In some examples, the electrosurgical tool 112 can include a collar 356 at a distal end of the handle 124. The collar 356 can be rotatable relative to the handle 124 to increase and/or decrease friction between an outer surface of the shaft 126 and an inner surface of the collar 356. In this way, the collar 356 to allow and/or inhibit (i) axial movement of the shaft 126 relative to the handle 124, and/or (ii) rotation of the shaft 126 and the monopolar electrosurgical electrode 128 relative to the handle 124.

As described above, in some examples, the shaft 126 can be rotatable relative to the handle 124 while one or more components in the interior bore 254 of the shaft 126 remain rotationally fixed relative to the handle 124. For instance, as described in further detail below, the electrosurgical tool 112 can further include smoke evacuation channel 148, the light sources 140 and the optical structure 142, and these components can be rotationally fixed relative to the handle 124. Providing for rotation of the monopolar electrosurgical electrode 128 together with the shaft 126 while rotationally fixing the smoke evacuation148, the light sources 140, and/or the optical structure 142 can help to simplify the design and/or reduce a cost of manufacture for the electrosurgical tool 112.

In some examples, the rotational arrangement of these components of the electrosurgical tool 112 can be achieved, at least in part, as a result of the monopolar electrosurgical electrode 128 extending distally from the distal end 126B of the shaft 126 such that (i) the shaft 126 conducts electrosurgical energy to the monopolar electrosurgical electrode 128, and (ii) rotation of the shaft 126 relative to the handle 124 causes corresponding rotation of the monopolar electrosurgical electrode 128 relative to the handle 124. For instance, at least a portion of the shaft 126 can be formed of an electrically conductive material such that the shaft 126 can supply the electrosurgical energy to the monopolar electrosurgical electrode 128.

In one example, the monopolar electrosurgical electrode 128 and the shaft 126 are formed as a single-part, monolithic structure. This can be beneficial in an implementation in which the monopolar electrosurgical electrode 128 is permanently fixed to the shaft 126 such that the monopolar electrosurgical electrode 128 cannot be replaced with another monopolar electrosurgical electrode 128. In another example, the monopolar electrosurgical electrode 128 and the shaft 126 can be separate components that are coupled to each other (e.g., by welding, soldering, and/or a friction fit coupling). In some implementations in which the monopolar electrosurgical electrode 128 and the shaft 126 are separate components, the monopolar electrosurgical electrode 128 can be removable from the shaft 126 and replaced with another monopolar electrosurgical electrode 128. In other implementations, the monopolar electrosurgical electrode 128 can be permanently fixed to the shaft 126 such that the monopolar electrosurgical electrode 128 cannot be replaced with another monopolar electrosurgical electrode 128.

In Figures 3-4, the shaft 126 includes an electrically conductive portion 126C and an insulator portion 126D. As noted above, the monopolar electrosurgical electrode 128 can extend from the electrically conductive portion 126C of the shaft 126. The insulator portion 126D of the shaft 126 can be a sleeve structure that covers an interface between the monopolar electrosurgical electrode 128 and the electrically conductive portion 126C of the shaft 126. In this arrangement, the insulator portion 126D can help to mitigate arcing and/or help to supply the electrosurgical energy to the monopolar electrosurgical electrode 128. Additionally, the shaft 126 can include a layer of insulator material 126E (e.g., a heat shrink material) covering a remainder of the electrically conductive portion 126C of the shaft 126 (e.g., a portion that is not covered by the insulator portion 126D of the shaft 126) to mitigate arcing and/or help to supply the electrosurgical energy to the monopolar electrosurgical electrode 128.

As described above, the printed circuit board 132 is electrically coupled to the shaft 126 to provide at least a portion of the electrical circuit for supplying the electrosurgical energy from the power cord 122 to the monopolar electrosurgical electrode 128. As shown in Figure 4, the printed circuit board 132 includes a first end 132A in the interior cavity 252 of the handle 124, a second end 132B in the interior bore 254 of the shaft 126, and a transmission portion extending between the first end 132A and the second end 132B of the printed circuit board 132.

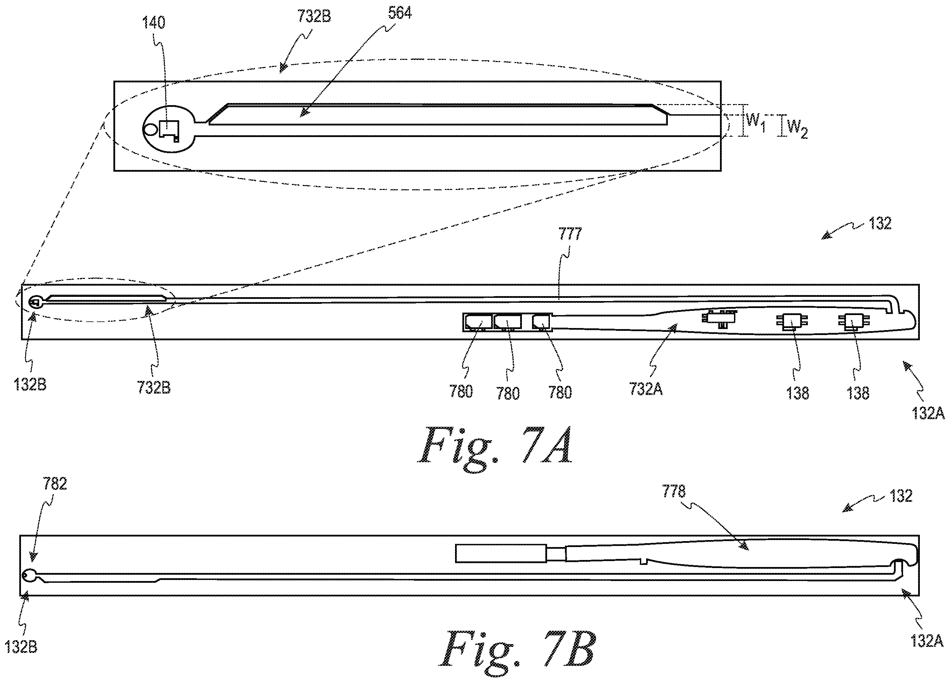

The first end 132A can be fixedly coupled to the handle 124 such that the first end 132A of the printed circuit board 132 does not move axially and/or rotationally relative to the handle 124. In Figure 4, a proximal section of the printed circuit board 132 (e.g., a section of the printed circuit board 132 that is nearer to the first end 132A than the second end 132B) includes the switches 138 (shown in Figure 1) and is fixedly coupled to the handle 124 adjacent to the user input devices 130. The proximal section of the printed circuit board 132 is described in further detail below with respect to an example shown in Figures 7A-7B.

The second end 132B of the printed circuit board 132 can be fixedly coupled to at least one component in the interior bore 254 of the shaft 126. For example, in Figure 3, the second end 132B of the printed circuit board 132 includes a LED PCB, which is fixedly coupled to a heatsink 458. This can provide for the printed circuit board 132 also supplying electrical energy from the DC power source 144 to the light sources 140. In this example, the light sources 140 are configured to move axially with the shaft 126 relative to the handle 124, but remain rotationally fixed relative to the handle 124 while the shaft 126 is rotatable relative to the handle 124.

In this arrangement, the transmission portion of the printed circuit board 132 can have a length that is equal to or greater than a distance between the first end 132A and the second end 132B of the printed circuit board 132 when the shaft 126 is in a distalmost (e.g., fully extended) position relative to the handle 124. When the shaft 126 is in a position that is proximal of the distalmost position relative to the handle, a slack of the transmission portion can accumulate in the interior bore 254 of the shaft 126 and/or the interior cavity 252 of the handle 124. For instance, the printed circuit board 132 can be a flexible printed circuit board such that the transmission portion can flex, bend, and/or fold back on itself to occupy an open space in the interior bore 254 of the shaft 126 and/or the interior cavity 252 of the handle 124 as the shaft 126 moves proximally and/or distally relative to the handle 124. Additionally, for instance, the transmission portion can twist and/or spiral in the open space response to the shaft 126 rotating relative to the handle 124.

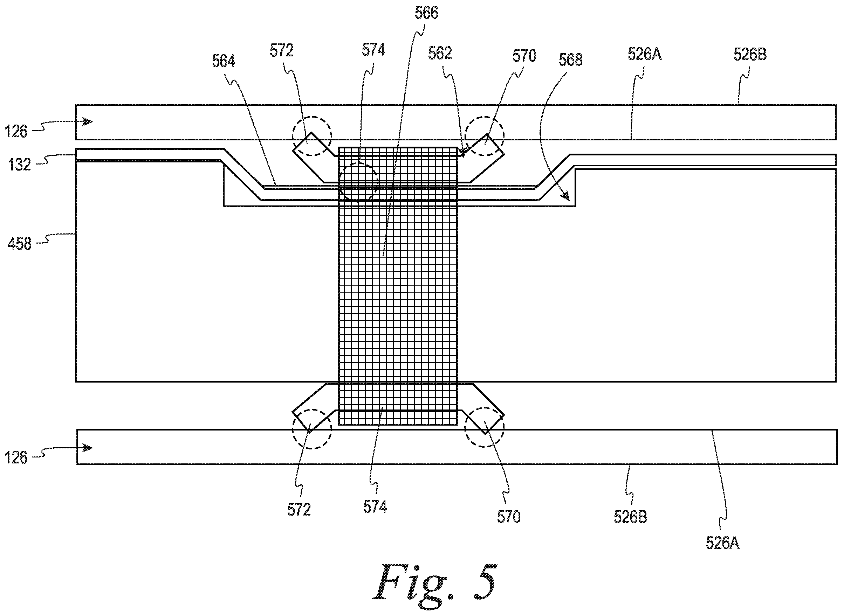

As described above, the electrosurgical tool 112 includes one or more components that can help to maintain the electrical coupling between the printed circuit board 132 and an interior surface of the shaft 126 (and, thus, the monopolar electrosurgical electrode 128. In Figures 3-4, a distal section of the printed circuit board 132 (e.g., a section of the transmission portion that is in the interior bore 254 and is distal of the proximal section of the printed circuit board 132) is maintained in electrical communication with the interior surface of the shaft 126 at least in part by the heatsink 458 in the interior bore 254 of the shaft 126. In addition to assisting to transfer heat away from the light sources 140, the heatsink 458 can provide an anchor point that can work with one or more additional components to electrically couple the printed circuit board 132 to the shaft 126. Figures 5-6 depict implementations in which the heatsink 458 assists in electrically coupling the printed circuit board 132 to the interior surface of the shaft 126 according to some examples. However, in other examples, the concepts shown and described with respect to Figures 5-6 can be extended to apply to implementations that omit the heatsink 458 and instead include a different anchor structure in the interior bore 254 of the shaft 256.

Figure 5 depicts a partial cross-sectional view of the shaft 126 through a line 360 in Figure 3, according to one example. In Figure 5, the shaft 126 includes an interior surface 526A and an exterior surface 526B. At least the interior surface 526A is formed from one or more electrically conductive materials. In some examples, the exterior surface 526B and/or a wall of the shaft 126 extending between the interior surface 526A and the exterior surface 526B can also be formed for the one or more electrically conductive materials.

As shown in Figure 5, the heatsink 458 is in the interior bore 254 of the shaft 126, and the printed circuit board 132 is between the heatsink 458 and the shaft 126. Additionally, as shown in Figure 5, the electrosurgical tool 12 includes an electrical brush 562 that electrically couples the printed circuit board 132 to the interior surface 526A of the shaft 126. For instance, the electrical brush 562 can be between the printed circuit board 132 and the interior surface 526A of the shaft 126, and the electrical brush 562 can include an electrically conductive material (e.g., a metal such as copper) such that the electrical brush 562 electrically couples a conductive contact 564 of the printed circuit board 132 to the interior surface 526A of the shaft 126.

The heatsink 458, the printed circuit board 132, the electrical brush 562, and the interior surface 526A of the shaft 126 can have respective sizes and shapes such that the printed circuit board 132 can be in electrical communication with the interior surface 526A via the electrical brush 562 in all axial positions and/or rotational orientations of the shaft 126 relative to the handle 124. For example, the electrical brush 562 and the heatsink 458 can have respective sizes and shapes such that the printed circuit board 132 is forced by the electrical brush 562 toward the heatsink 458 to maintain the electrical coupling between the conductive contact 564 of the printed circuit board 132 and the electrical brush 562. For instance, the heatsink 458, the printed circuit board 132, and the electrical brush 562 can be configured such that the electrical brush 562 clamps the printed circuit board 132 to the heatsink 458. In Figure 5, the electrical brush 562 can extend around at least one half of a circumference of the heatsink 458 to help retain the electrical brush 562 in electrical communication with the conductive contact 564 of the printed circuit board 132.

Additionally, as shown in Figure 5, the electrosurgical tool 112 can also include an attachment member 566 that couples the electrical brush 562, the printed circuit board 132, and the heatsink 458 to each other. For instance, the attachment member 566 can extend around and applies a radially inward force to the electrical brush 562, the printed circuit board 132, and the heatsink 458. This can help to improve the contact between the conductive contact 564 of the printed circuit board 132 and the electrical brush 562, and/or help to fix a position of the electrical brush 562 relative to the heatsink 458 and the printed circuit board 132.

In one example, the attachment member 566 can be a band formed of a heat shrink material. In this example, a process for forming the electrosurgical tool 112 can include (i) positioning the attachment member 566 around an assembly of the heatsink 458, the printed circuit board 132, and the electrical brush 562, and (ii) applying heat to the attachment member 566 to shrink the attachment member 566 around and apply the radially inward force to the assembly. As other examples, the attachment member 566 can include at least one component selected from among a group consisting of: Kapton tape, an o-ring, and an adhesive. Also, in other example implementations, the attachment member 566 can be omitted.

In some examples, the heatsink 458 can include a recess 568 extending around at least a portion of a circumference of the heatsink 458, and at least a portion of the printed circuit board 132 can be in the recess 568 of the heatsink 458. For instance, in Figure 5, the conductive contact 564 is in the recess 568 of the heatsink 458. The recess 568 can help to mitigate the electrical brush 562 moving axially relative to the printed circuit board 132, the heatsink 458, and/or the shaft 126. Additionally, the recess 568 can help to provide additional space within the interior bore 254 of the shaft 126 for electrical brush 562.

To electrically couple the printed circuit board 132 to the shaft 126, (i) at least a portion of the electrical brush 562 is in electrical communication with the conductive contact 564 of the printed circuit board 132, and (ii) at least a portion of the electrical brush 562 is in electrical communication with the interior surface 526A of the shaft 126. As an example, in Figure 5, the electrical brush 562 includes a distal end 570, a proximal end 572, and a center portion 574 between the distal end 570 and the proximal end 572. The center portion 574 can engage the conductive contact 564, and the distal end 570 and the proximal end 572 can engage the interior surface 526A of the shaft 126.

In some examples, the center portion 574 can have a shape that corresponds to a shape of a conductive contact 564 of printed circuit board 132 (e.g., in the recess 568 of the heatsink 458). In one example, at least a portion of the recess 568 can define a substantially planar surface for engaging with the printed circuit board 132. The conductive contact 564 and a portion of the electrical brush 562 that engages the conductive contact 564 can also have a substantially planar surface. This can help to provide relatively uniform contact between the conductive contact 564 and the electrical brush 562. However, in other examples, the portion of the recess 568 that engages the printed circuit board 132 at the conductive contact 564, the conductive contact 564, and/or the electrical brush 562 can have a different shape (e.g., a curved shape).

As shown in Figure 5, in some examples, the distal end 570 of the electrical brush 562 and the proximal end 572 of the electrical brush 562 can extend radially outward from the center portion 574 to directly contact the interior surface 526A of the shaft 126. The distal end 5700 can also extend distally from the center portion 574 and the proximal end 572 can extend proximally from the center portion 574. In this arrangement, the electrical brush 562 can have a flexibility that is suitable to accommodate a force applied to the electrical brush 562 by (i) the shaft 126 on one side and (ii) the heatsink 458 and the printed circuit board 132 on the other side. Additionally or alternatively, this arrangement can help to reduce friction as the shaft 126 rotates relative to the electrical brush 562.

Within examples, the electrical brush 562 can extend around at least a portion of the circumference of the interior surface 562A of the shaft 126 (e.g., in a dimension that is perpendicular to the longitudinal axis 350 shown in Figure 3). For instance, the electrical brush 562 can extend around at least half of the circumference of the interior surface 526A of the shaft 126, and/or around an entirety of the circumference of the interior surface 526 of the shaft 126. This can beneficially provide for the electrical brush 562 electrically coupling the conductive contact 564 of the printed circuit board 132 to the shaft 126 at one or more locations around the circumference of the interior surface 526A of the shaft 126.

In some examples, at least a portion of the distal end 570 and at least a portion of the proximal end 572 of the electrical brush 562 can have a shape that matches a shape of the interior surface 562A of the interior surface 26A of the shaft 126 (e.g., at least a portion of the distal end 570 and/or the proximal end 572 can have a curved shape that matches a curved contour of the interior surface 526A). This can help to enhance an extent of contact between the electrical brush 562 and the shaft 126. In other examples, the distal end 570 and/or the proximal end 572 can have shapes that are different from the shape of the interior surface 526A so long as the electrical brush 562 is in electrical communication with the interior surface 526A of the shaft 126 at one or more positions on the interior surface 526A.

As described above, the distal end 570 and/or the proximal end 572 of the electrical brush 562 can define an outer surface of the electrical brush 562 for engaging the interior surface 526A of the shaft 126, whereas the center portion 574 of the electrical brush 562 can define an inner surface of the electrical brush 562 for engaging the conductive contact 564 of the printed circuit board 132. However, in other examples, the electrical brush 562 can be configured differently. For instance, in one other example, (i) at least one of the distal end 570, the proximal end 572, and the center portion 574 can define the outer surface of the electrical brush 562, and (ii) at least another one of the distal end 570, the proximal end 572, and the center portion 574 can define the inner surface of the electrical brush 562.

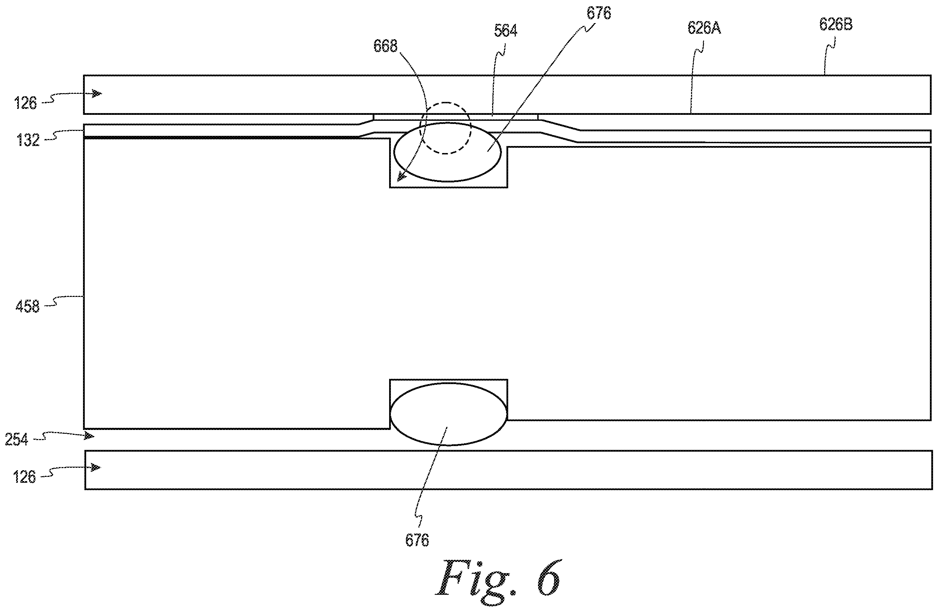

Referring now to Figure 6, another example implementation in which the heatsink 458 assists in electrically coupling the printed circuit board 132 to the interior surface of the shaft 126. Figure 6 also depicts a partial cross-sectional view of the shaft 126 through the line 360 in Figure 3, according to another example. In Figure 6, the shaft 126 includes an interior surface 626A and an exterior surface 626B. At least the interior surface 626A is formed from one or more electrically conductive materials. In some examples, the exterior surface 626B and/or a wall of the shaft 126 extending between the interior surface 626A and the exterior surface 626B can also be formed for the one or more electrically conductive materials.

In Figure 6, the electrosurgical tool 112 includes a biasing member 676, which applies a force to printed circuit board 132 to press the conductive contact 564 of the printed circuit board 132 into electrical contact with the interior surface 626A of the shaft 126. For example, in Figure 6, the biasing member 676 is between the heatsink 458 and the printed circuit board 132 such that a first portion of the biasing member 676 can contact the printed circuit board 132, and a second portion of the biasing member 676 can contact the heatsink 458 to force the conductive contact 564 of the printed circuit board 132 in a radially outward direction towards the interior surface 526A of the shaft 126.

Within examples, the biasing member 676 can have a size and/or a shape relative to respective sizes and/or shapes of the heatsink 458, the printed circuit board 132, and the shaft 126 such that the biasing member 676 presses the conductive contact 564 against the interior surface 626A of the shaft 126 with sufficient force to maintain electrical communication while the shaft 126 rotates relative to the handle 124 and/or the printed circuit board 132.

In some examples, the biasing member 676 can include an elastomeric material. This can allow the biasing member 676 to compress and/or expand for easy assembly while maintaining contact within a tolerance range of the shaft 126. Additionally or alternatively, forming the biasing member 676 from the elastomeric material can help to inhibit ingress of fluids. In one example, the basing member 676 can include an O-ring.

In some examples, the biasing member 676 can extend around the circumference of the heatsink 458 and provide a gasket that inhibits ingress of fluid in a space between the interior surface 626A of the shaft 126 and the heatsink 458. For instance, at an axial position of the biasing member 676 along the longitudinal axis 350, the space between the interior surface 626A of the shaft 126 and the heatsink 458 can be substantially occupied by (i) a combination of the printed circuit board 132 and the biasing member 676 along a first circumferential portion of the space, and (ii) the biasing member 676 along a second circumferential portion of the space. In examples in which the biasing member 676 provides the gasket, the biasing member 676 can inhibit foreign matter (e.g., fluid and/or debris) from traveling proximally along the shaft 126 and into the interior cavity 252 of the handle 124 (e.g., outside of the smoke evacuation channel 148 and the smoke evacuation chamber 152, if included). This can help mitigate such foreign debris contacting electrical components within the handle 124 (e.g., a proximal portion of the printed circuit board 132).

In some examples, the heatsink 458 can include a recess 668 extending around at least a portion of a circumference of the heatsink 458, and the biasing member 676 can be in the recess 668. The recess 668 can help to retain to maintain the biasing member 676 in a fixed axial position (e.g., along the longitudinal axis 350) relative to the heatsink 458. As such, the recess 668 can help to mitigate the biasing member 676 moving axially relative to the printed circuit board 132, the heatsink 458, and/or the shaft 126. Additionally, the recess 668 can help to provide additional space within the interior bore 254 of the shaft 126 for the biasing member 676.

In some examples, the recess 668 can also extend around an entire circumference of the heatsink 458. For instance, in an example in which the biasing member 676 extends around the entire circumference of the heatsink 458, the recess 668 can extend around the entire circumference of the heatsink 458 to help retain the biasing member 676 in the fixed axial position. However, in other examples, the recess 668 can extend around less than an entirety of the circumference of the heatsink 458. This may, for instance, help to reduce cost by improving an assembly process and/or by reducing a cost for machining.

Referring now to Figures 7A-7B, an implementation of the printed circuit board 132 is shown according to an example. In particular, Figure 7A depicts a first side view of the printed circuit board 132, and Figure 7B depicts a second side view of the printed circuit board 132 that is opposite of the first side view shown in Figure 7A.

In Figures 7A-7B, the printed circuit board 132 is a flexible printed circuit board. For example, the printed circuit board 132 can include a plurality of electrical circuits on a flexible substrate. In one implementation, for instance, the printed circuit board 132 can include one or more conductive layers of traces of an electrically conductive material (e.g., copper) on a polyimide dielectric layer.

As shown in Figures 7A-7B, the printed circuit board 132 has the first end 132A, the second end 132B, and a transmission portion 777 extending between the first end 132A and the second end 132B. As described above, the first end 132A can be fixedly coupled to the handle 124 in the interior cavity 252 of the handle 124 such that the first end 132A of the printed circuit board 132 does not move axially and/or rotationally relative to the handle 124. For instance, in Figure 7A, a proximal section 732A of the printed circuit board 132 includes the switches 138 and is fixedly coupled to the handle 124 adjacent to the user input devices 130. The switches 138 are actuatable by the user input devices 130 (e.g., a plurality of buttons 330A, 330B shown in Figure 3) on the handle 124 to control the electrosurgical energy supplied to the monopolar electrosurgical electrode 128.

As shown in Figure 7B, the printed circuit board 132 can include a first PCB stiffener 778 coupled to the proximal section 732A on the second side of the printed circuit board 132 to assist in fixedly coupling the proximal section 732A to the handle 124. The first PCB stiffener 778 can be formed from any material suitable to increase a stability and/or a rigidity of the proximal section 732A of the printed circuit board 132. As one example, the first PCB stiffener 778 can include a thermally conductive material such as, for instance, aluminum. This can additionally help to transfer heat away from the proximal section 732A of the printed circuit board 132.

The proximal section 732A of the printed circuit board 132 can also include a plurality of power cord contacts 780 for electrically coupling to respective conductors of the power cord 122 (shown in Figure 1). In Figure 7A, the printed circuit board 132 includes three power cord contacts 780 configured to couple to three conductors of the power cord 122 that provide for supplying the electrosurgical energy from the electrosurgical generator 110 to the printed circuit board 132. In an example in which the DC power source 144 is located along the power cord 122 and/or at a plug of the power cord 122, the printed circuit board 132 can include two power cord contacts 780 configured to couple to two conductors of the power cord 122 that provide the DC power from the DC power source 144 to the light sources 140.

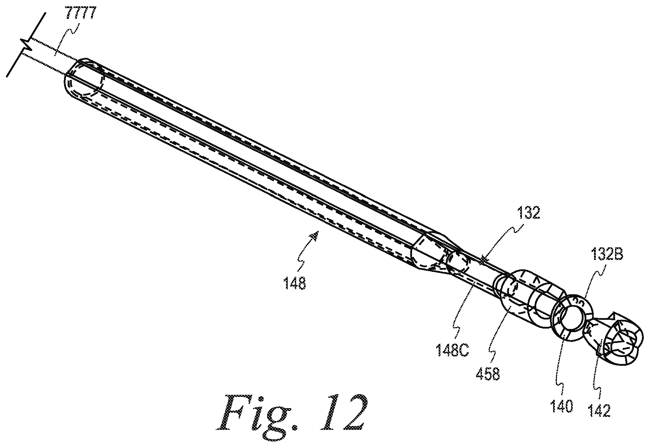

As described above, the second end 132B of the printed circuit board 132 can be fixedly coupled to at least one component in the interior bore 254 of the shaft 126 (e.g., the heatsink 458). In the example shown in Figures 3-4 and 7A-7B, the second end 132B of the printed circuit board 132 includes the light source(s) 140, and is fixedly coupled between the heatsink 458 and the optical structure 142. As shown in Figure 7B, printed circuit board 132 can include a second PCB stiffener 782 coupled to the second end 132B on the second side of the printed circuit board 132 to assist in fixedly coupling the second end 132B between the heatsink 458 and the optical structure 142. The second PCB stiffener 782 can be formed from any material suitable to increase a stability and/or a rigidity of the second end 132B and/or the light sources 140. As one example, the second PCB stiffener 782 can include a thermally conductive material such as, for instance, aluminum. This can additionally help to transfer heat away from the light sources 140 and towards the heatsink 548. The coupling of the second end 132B to the heatsink 548 and/or the optical structure 142 is described in further detail below with respect to Figures 12-13.

The transmission portion 777 extends between the first end 132A and the second end 132B. As shown in Figures 4 and 7A-7B, the printed circuit board 132 is elongated in an axial dimension extending between the first end 132A and the second end 132B, and the axial dimension can be parallel to the longitudinal axis 350 (shown in Figure 3) of the shaft 126. In this arrangement, the transmission portion 777 of the printed circuit board 132 can have a length that is equal to or greater than a distance between the first end 132A and the second end 132B of the printed circuit board 132 when the shaft 126 is in a distalmost (e.g., fully extended) position relative to the handle 124. When the shaft 126 is in a position that is proximal of the distalmost position relative to the handle, a slack of the transmission portion can accumulate in the interior bore 254 of the shaft 126 and/or the interior cavity 252 of the handle 124.

For instance, in Figures 4-6, the heatsink 458 does not extend to a proximal end of the interior bore 254 such that a space is provided in the interior bore 254 between the heatsink 458 and the proximal end of the shaft 126. This space can thus accommodate the slack of the printed circuit board 132, which facilitates axial movement of the shaft 126 and the second end 132B of the printed circuit board 132 relative to the first end 132A of the printed circuit board 132.

As shown in Figure 7A, the printed circuit board 132 includes the conductive contact 564 for electrically coupling the printed circuit board 132 to the interior surface 526A, 626A of the shaft 126, and the conductive contact 564 is between the first end 132A and the second end 132B of the printed circuit board 132. The conductive contact 564 can include a portion of a conductive trace of the printed circuit board 132 that is exposed and not covered by a dielectric material. The exposed conductive material of the conductive contact 564 can thus electrically couple to the interior surface 526A, 626A of the shaft 126 as described above. As one example, the conductive contact 564 can include copper.

As shown in Figure 7A, a width W₁ of the printed circuit board 132 at the conductive contact 564 can be greater than a width W₂ of the printed circuit board 132 at a first section of the printed circuit board 132 that is proximal of the conductive contact 564 and/or second section of the printed circuit board 132 that is distal of the conductive contact 564. In this arrangement, the conductive contact 564 can be enlarged relative to one or more sections of the transmission portion 777 to increase a surface area for electrical contact with the interior surface 526A, 626A of the shaft 126, whereas the proximal and/or distal sections of the transmission portion 777 can have a relatively smaller size to address space constraint considerations due to accumulation of the slack in the transmission portion 777 at one or more axial positions of the shaft 126 relative to the handle 124.

In this arrangement, the first end 132A of the printed circuit board 132 can receive the electrosurgical energy from the power cord 122 (shown in Figure 1), the transmission portion 777 can supply the electrosurgical energy from the first end 132A to the conductive contact 564, the conductive contact 564 can supply the electrosurgical energy to the interior surface 526A, 626A of the shaft 126, and the shaft 126 can supply the electrosurgical energy to the monopolar electrosurgical electrode 128. Within examples, the printed circuit board 132 can supply the electrosurgical energy in this manner in all axial positions and/or in all rotational orientations of the shaft 126 relative to the handle 124. Additionally, in examples that include the light sources 140, the first end 132A of the printed circuit board 132 can receive the DC power from the power cord 122 (shown in Figure 1), and the transmission portion 777 can supply the DC power from the first end 132A to the light sources 140 at the second end 132B.

As described above, the printed circuit board 132 can include the first PCB stiffener 778 at the proximal section 732A and/or the second PCB stiffener 782 at the second end 132B of the printed circuit board. Within examples, at least part of or an entirety of the transmission portion 777 of the printed circuit board can omit a PCB stiffener. This can allow the transmission portion 777 to have a greater flexibility than the second end 132B and/or the proximal section 732A, which in turn can allow for the transmission portion 777 to flex and/or bend when accommodating movement of the shaft 126 relative to the handle 124.

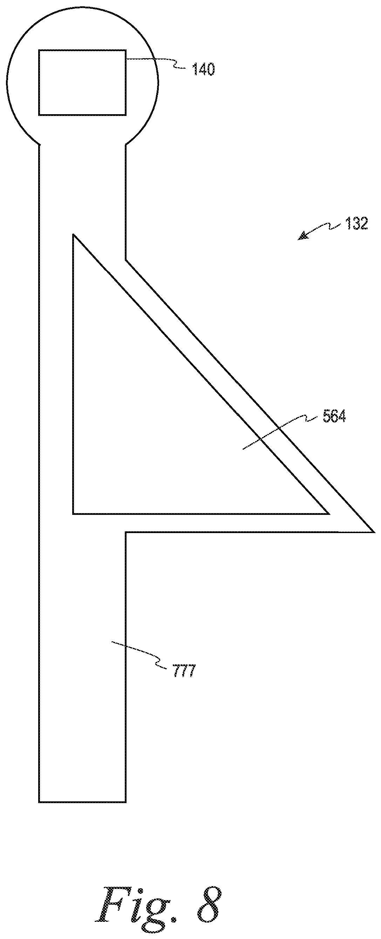

Figure 8 depicts another implementation for the printed circuit board 132 according to another example. In particular, Figure 8 depicts as distal section of the printed circuit board 132, the second end 132B, the light source 140, and the conductive contact 564 according to the example. As shown in Figure 8, the transmission portion 777 can have a rectangular shape, and the conductive contact 564 can have a triangular shape. The triangular shape of the conductive contact 564 can help to increase a surface area for electrical contact.

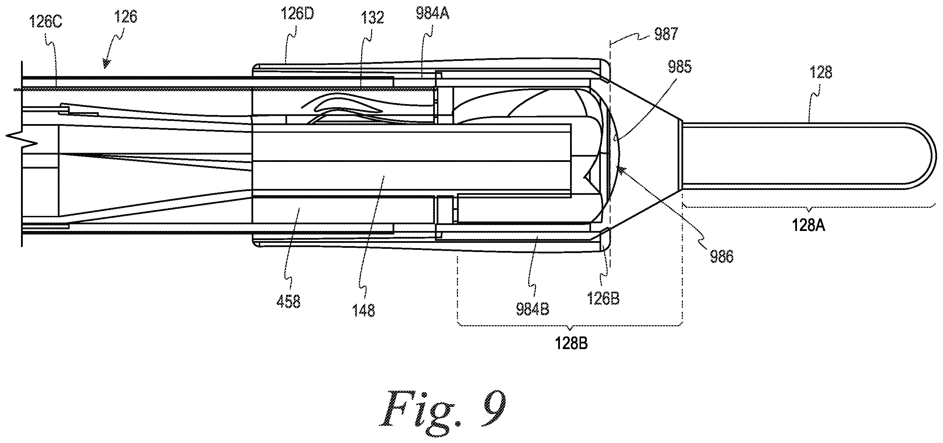

Referring now to Figure 9, an enlarged view of the cross-section of the distal end 126B of the shaft 126 and the monopolar electrosurgical electrode 128 taken through the longitudinal axis 350 is shown according to an example. As shown in Figure 9, a distal portion 128A of the monopolar electrosurgical electrode 128 can define a working end that is configured to apply electrosurgical energy to tissue. A proximal portion 128B of the monopolar electrosurgical electrode 128 can include a first leg 984A extending from a distal end 126B of the shaft 126, and a second leg 984B extending from the distal end of the conductive portion 126C of the shaft 126.

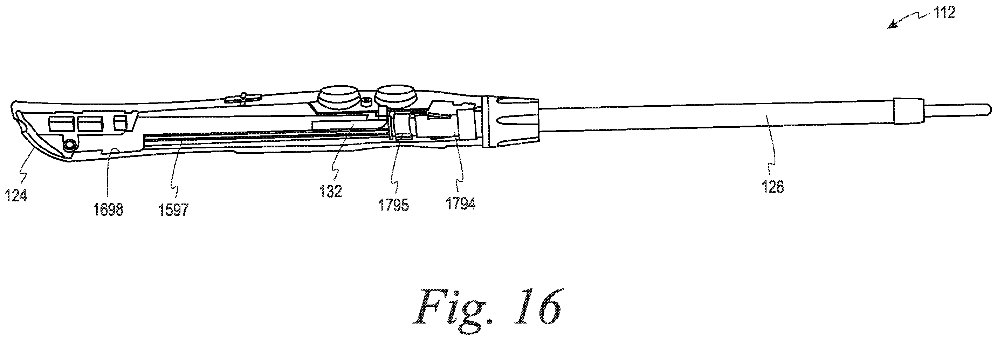

In Figure 16, the first leg 984A and the second leg 984B are diametrically opposed to each other around a circumference of the distal end 126B of the shaft 126. Additionally, a proximal-facing surface 985 of the proximal portion 128B of the monopolar electrosurgical electrode 128 can taper, along a distal direction, toward a center axis of the shaft 126 to define a gap 986 between the proximal-facing surface 985 and a plane 987 at a distalmost end of the shaft 126. The gap 986 can help to improve air flow and suction at the distal end 126B of the shaft 126.

Additionally, as shown in Figure 9, the sleeve structure of the insulator portion 126D can extend around the optical structure142. The sleeve structure may be an optically opaque material. To enhance an intensity of the light emitted from the optical structure 142, at least a portion of each distal transmission surface the optical structure 142 can extend to a position distal of a distal-most end of the sleeve structure of the insulator portion 126D.

As noted above, the monopolar electrosurgical electrode 128 can include a proximal portion 128B extending from the distal end of the shaft 126, and a distal portion 128A that comprises a working end configured to apply electrosurgical energy to tissue. In Figures 3-4 and 9, a center axis of the distal portion 128A of the monopolar electrosurgical electrode 128 and a center axis of the smoke evacuation channel 148 are collinear. In this arrangement, the smoke evacuation channel 148 can have a substantially constant size around a circumference of the monopolar electrosurgical electrode 128. This can help to provide relatively consistent suction each point around the monopolar electrosurgical electrode 128. However, in other examples, the center axes of the monopolar electrosurgical electrode 128 and the shaft 126 can be offset and parallel relative to each other.

Additionally, as shown in Figures 3-4 and 9, the smoke evacuation channel 148 can define a space that is void of any other structure between a proximal end 148A of the smoke evacuation channel 148 and a distal end 148B of the smoke evacuation channel 148. This can provide for more efficiently using the relatively limited size of the interior bore 254 to enhance suction via the smoke evacuation channel 148 as compared to other implementations in which the monopolar electrosurgical electrode 128 and/or other components are disposed in the smoke evacuation channel 148.

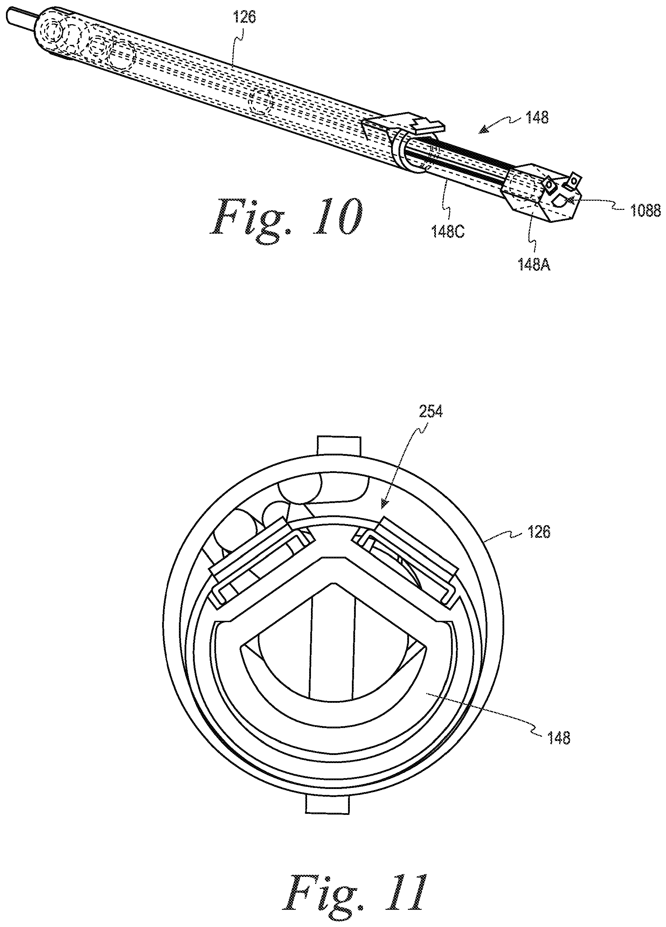

Additionally, as noted above, the smoke evacuation channel 148 can be rotationally fixed relative to the handle 124 such that the shaft 126 and the monopolar electrosurgical electrode 128 are rotatable relative to the smoke evacuation channel 148 according to some examples. Figures 10-11 depict the smoke evacuation channel 148 and the shaft 126 according to an example. As shown in Figures 4 and 10-11, at least a portion of the smoke evacuation channel 148 can have a non-circular shape to inhibit rotation of the smoke evacuation channel 148 relative to the handle 124 while the shaft 126 and the monopolar electrosurgical electrode 128 rotate relative to the handle 124.

For example, the proximal end 148A of the smoke evacuation channel 148 can include a non-rotational fitting that is configured to engage with a correspondingly shaped structure in the handle 124, and the non-rotational fitting can have a non-circular cross-sectional shape. In Figures 4 and 10, for example, the proximal end 148A of the smoke evacuation channel 148 has a hexagonal feature that engages a hexagonally shaped socket formed in an interior surface of the handle 124 to prevent rotation between the smoke evacuation channel 148 and the handle 124. Additionally, as shown in Figure 11, a gap can be defined between the shaft 126 and the smoke evacuation channel 148 to provide for rotation of the shaft 126 relative to the smoke evacuation channel 148.

As shown in Figure 10, the non-rotational fitting at the proximal end 148A of the smoke evacuation channel 148 can include a through-bore 1088 that has a cross-sectional area that is less than a cross-sectional area of a body 148C of the smoke evacuation channel 148, which is proximal of the non-rotational fitting. The relatively smaller size of the through-bore 1088 can assist in directing the smoke into a relatively smaller volume of space as the smoke exits the proximal end 148A of the smoke evacuation channel 148. This can beneficially help to reduce or prevent exposing electrical components in the interior bore 125 of the handle 124 to the smoke.

Figure 12 depicts a partially exploded view of an assembly of the printed circuit board 132, the light source 140, the optical structure 142, the heat sink 584, and the smoke evacuation channel 148 according to an example. As shown in Figures 4, 9, and 12, the electrosurgical tool 112 can include the light sources 140 and the optical structure 142 in the shaft 126 of the housing 123. As noted above, the light sources 140 are configured to emit light into the optical structure 142, and the optical structure 142 is configured to transmit the light from the light sources 140 in a distal direction and emit the light from the distal end 126B of the shaft 126.

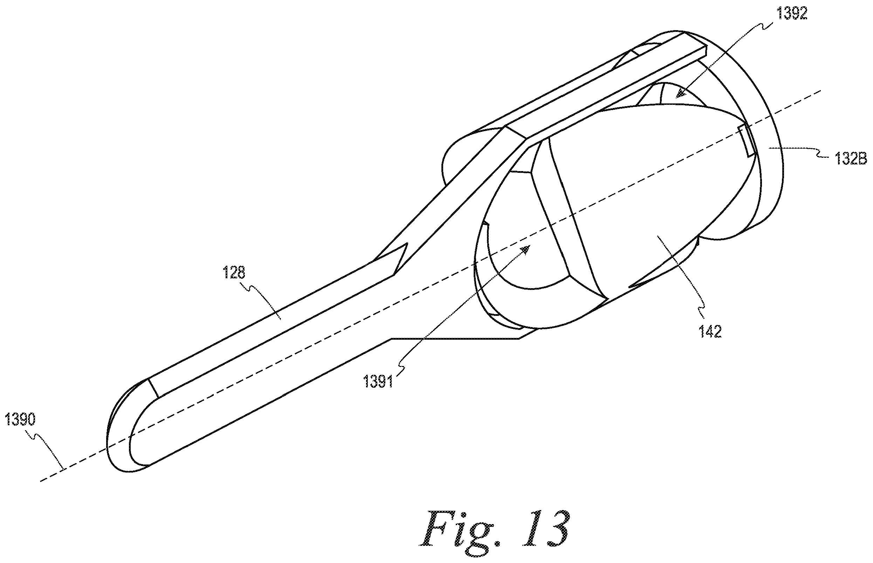

In Figure 12, the light sources 140 are on the second end 132B of the printed circuit board 132. The transmission portion 777 can extend along the smoke evacuation channel 148 and the heatsink 458. In an example, the second end 132B of the printed circuit board 132 can be fixedly coupled by a washer (e.g., a star washer) that is pressed against a proximal end of the heatsink 458 to force the heatsink 458, the second end 132B (e.g., including the second PCB stiffener 782 and the light sources 140), and the optical structure 142 to mate with the first leg 984A and/or the second leg 984B of the monopolar electrosurgical electrode 128. For instance, Figure 13 depicts the mating between the first leg 984A and the second leg 984B with the monopolar electrosurgical electrode 128. In this arrangement, the second end 132B can be compressed and fixedly held in place by the heatsink 458 and the optical structure 142.

Figure 13 depicts an assembly of the optical structure 142 and the monopolar electrosurgical electrode 128, according to an example. As shown in Figure 13, the electrosurgical electrode 128 has a longitudinal axis 1390 extending between the proximal end of the monopolar electrosurgical electrode 128 and a distal end of the monopolar electrosurgical electrode 128. The light sources 140 can be arranged circumferentially around the longitudinal axis 1390 of the monopolar electrosurgical electrode 128. Arranging the light sources 140 around the monopolar electrosurgical electrode 128 can help to distribute the light around the entire circumference of the monopolar electrosurgical electrode 128, which can help to mitigate shadows and provide greater uniformity of illumination in all rotational alignments of the monopolar electrosurgical electrode 128 relative to the handle 124 and/or the electrosurgical tool 112 relative to the target tissue.

Additionally, the smoke evacuation channel 148 can extend through an aperture 1391 in the optical structure 142 and a PCB aperture 1392 in the second end 132B of the printed circuit board 132. This can help to locate the smoke evacuation channel 148 at a center of the shaft 126 (e.g., the center axis of the smoke evacuation channel 148 and the center axis of the shaft 126 can be collinear), which can enhance suction at the surgical site. In this arrangement, the electrosurgical tool 112 can provide illumination and suction around the circumference of the monopolar electrosurgical electrode 128. Further, integrating the optical structure 142 in this arrangement can improve a quality of illumination, reduce a size of the distal end of the electrosurgical tool, and improve a line of sight to the surgical site.

Referring back to Figure 3, the user input device(s) 130 can include a first button 330A and a second button 330B on an exterior surface of the handle 124. In one implementation, the first button 330A can be actuated to operate the electrosurgical tool 112 in a cutting mode of operation, and the second button 330B can be actuated to operate the electrosurgical tool 112 in a coagulation mode of operation. In this example, a third button (not shown) can be provided on the plug of the power cord 122 and/or on the electrosurgical generator 110, and the third button can be actuated to operate the light source 140 (i.e., to cause the light source 140 to emit light or cease emitting light). As described above, the user input device(s) 130 can be configured differently in other examples. For instance, the electrosurgical tool 112 can be operable in a lesser quantity of modes of operation, a greater quantity of modes of operation, and/or different types of modes of operation in other examples (e.g., such as the example modes of operation described above). Additionally, for instance, the at least one user input device 130 can additionally or alternatively include the user interface 116 of the electrosurgical generator 110 and/or another external device (e.g., a footswitch) for operating the electrosurgical tool 112 in one or more modes of operation. Also, for instance, the user input devices 130 on the handle 124 can include the third button for operating the light source 140.

Although the electrosurgical tool 112 shown in Figures 3-13 includes the smoke evacuation channel 148, the light sources 140, and the optical structure 142, the electrosurgical tool 112 can omit one or more of these components in other implementations.

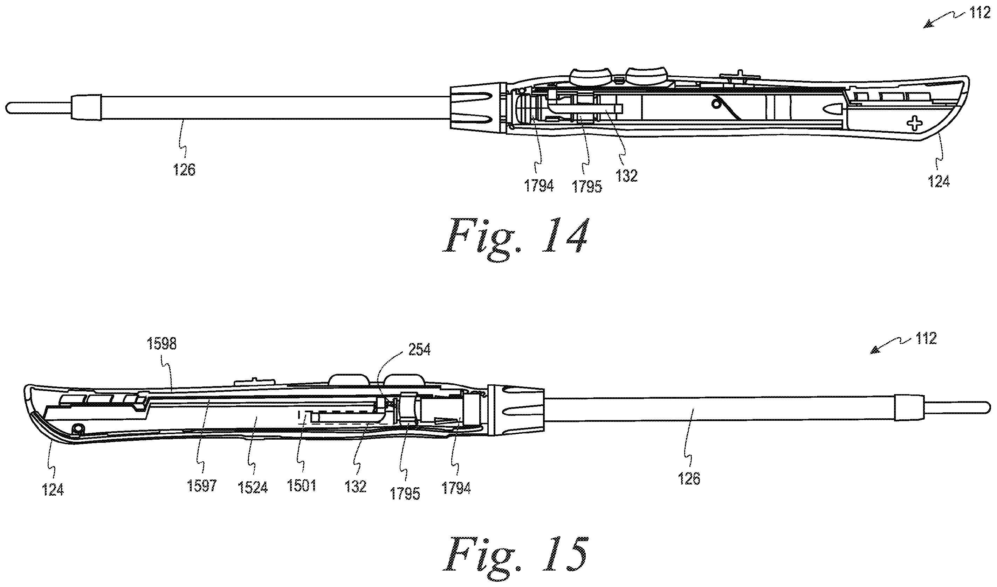

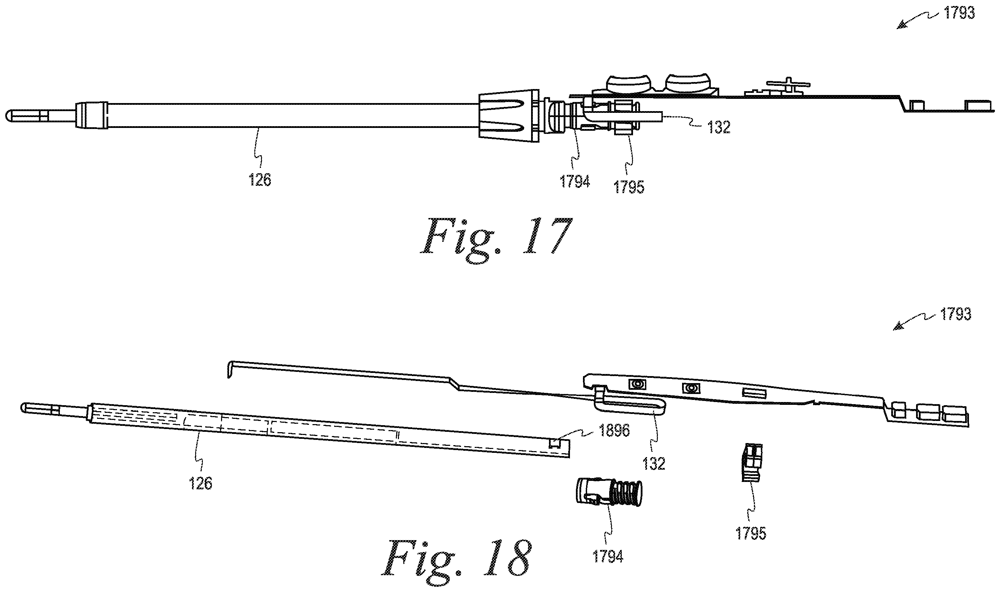

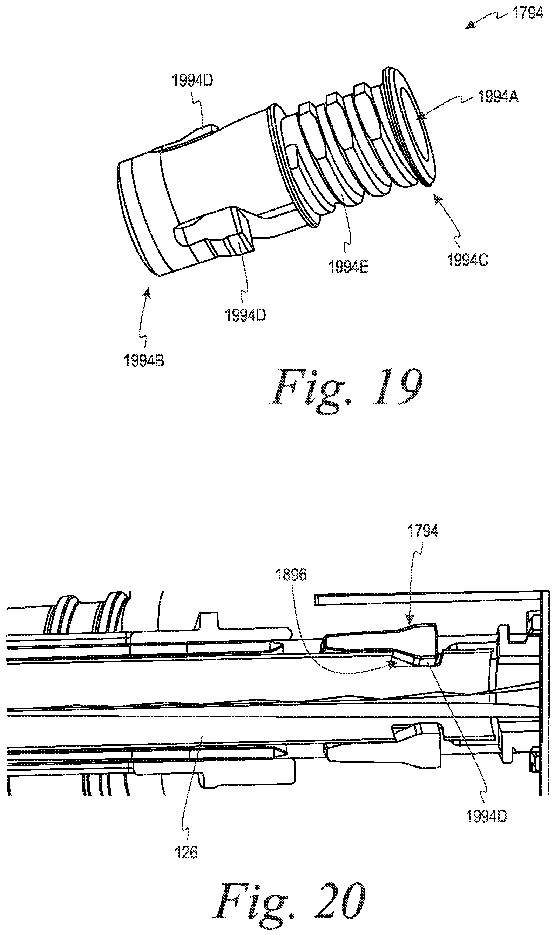

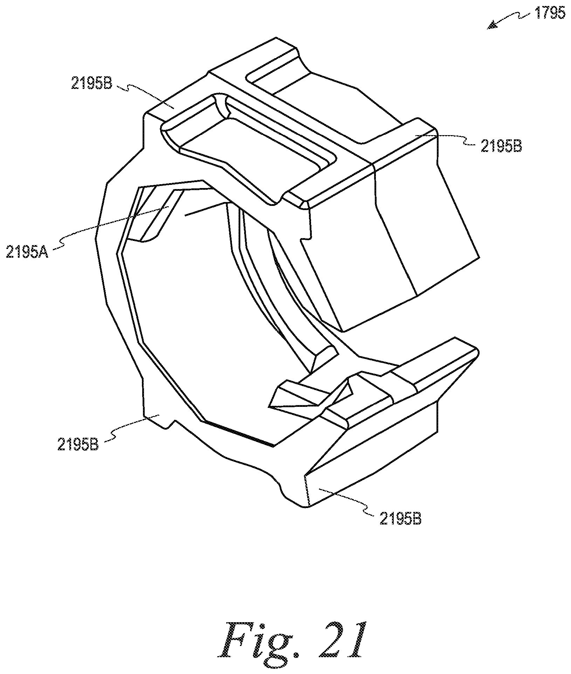

Figures 14-21 depicts additional components of the electrosurgical tool 112, according to additional or alternative examples. In particular, Figure 14 depicts a side view of the electrosurgical tool 112 with a first portion of the handle 124 omitted for illustration purposes, Figure 15 depicts a first perspective view of the electrosurgical tool 112 with a second portion of the handle 124 (opposite the first portion of the handle 124) omitted for illustration purposes, Figure 16 depicts a second perspective view of the electrosurgical tool 112 with the second portion of the handle 124 omitted for illustration purposes, Figure 17 depicts the electrosurgical tool 112 with both the first portion and the second portion of the handle 124 omitted for illustration purposes, Figure 18 depicts an exploded view of a stop assembly 1793 of the electrosurgical tool 112, Figure 19 depicts a shaft stop 1794 of the stop assembly 1793 according to an example, and Figure 20 depicts a partial cross-sectional view of a coupling between the shaft stop 1794 and the shaft 126, and Figure 21 depicts a rotation nut 1795 according to an example.

As shown in Figures 15-16, a portion of the printed circuit board 132 can be fixedly coupled to an internal wall 1524 of the handle 124. The portion of the printed circuit board 132 that is coupled to the internal wall 1524 of the handle 124 can be a portion of the printed circuit board 132 that is located outside of the interior bore 254 of the shaft 126. Fixedly coupling the printed circuit board 132 to the internal wall 1524 of the handle 124 can help to mitigate or prevent tangling and/or bunching of the printed circuit board 132 as the shaft 126 moves longitudinally relative to the handle 124.

As one example, Figure 15 indicates a zone 1501 of the internal wall 1524 of the handle 124 at which the printed circuit board 132 can be fixedly coupled. The zone 1501 can be elongated along the longitudinal axis 350 (shown in Figure 3). This can help to increase a surface area of the coupling between the printed circuit board 132 and the internal wall 1524 of the handle 124, which can enhance a strength of the coupling between the printed circuit board 132 and the internal wall 1524 of the handle 124.

In some examples, the zone 1501 can have a surface roughness that is greater than a surface roughness of another portion of the internal wall 1524 of the handle 124 surrounding the zone 1501. For instance, in one implementation, a manufacturing process can include roughening the internal wall 1524 at the zone 1501 (e.g., via sanding, thermal energy, chemical etching, and/or abrasive blasting), and not roughening the other portion of the internal wall 1524. In another implementation, the manufacturing process can include reducing a roughness of the other portion of the internal wall 1524 and maintaining the surface roughness of the zone 1501. By providing the zone 1501 with an increased surface roughness relative to the other portion of the internal wall 1524, the zone 1501 can enhance coupling the printed circuit board 132 and the handle 124 and/or the zone 1501 can provide a tactile indication of a suitable coupling location to an assembler during the manufacturing process.

In the example shown in Figures 14-20, the electrosurgical tool 112 is configured to define a finite range of rotation between the shaft 126 and the handle 124. Limiting the range of rotation between the shaft 126 and the handle 124 can help to mitigate excessive stress on the printed circuit board 132 due to over-rotation of the shaft 126 relative to the handle 124. As noted above, in one implementation, the monopolar electrosurgical electrode 128 can be rotatable by more than 360 degrees relative to the handle 124. This can improve an ease of use by allowing an operator to freely rotate the monopolar electrosurgical electrode 128 without limitation. However, in other implementations, the monopolar electrosurgical electrode 128 can be rotatable by less than or equal to 360 degrees (e.g., rotatable by 180 degrees or rotatable by 360 degrees). This may still allow an operator to achieve a desired rotational arrangement, but with the possibility that the operator may rotate in first direction, reach a stop limiting further rotation, and then rotate back in a second direction to achieve the desired rotational arrangement.

In the example shown in Figures 14-20, the electrosurgical tool 112 includes a stop assembly 1793 that is configured to limit a range of rotational movement between the shaft 126 and the handle 124 to a predefined range of rotational movement. For example, in Figures 14-20, the stop assembly 1793 is configured to provide the shaft 126 and the handle 124 with a range of rotational motion of approximately 400 degrees relative to each other. However, as described above, the stop assembly 1793 can be configured to allow the shaft 126 and the handle 124 to have a different range of rotational motion relative to each other.

As shown in Figures 17-18, the stop assembly 1793 includes the shaft 126, the shaft stop 1794, and a rotational nut 1795. In this example, the shaft stop 1794 is fixedly coupled to a proximal portion of shaft 126, and the rotational nut 1795 is rotatably coupled to the shaft stop 1794. As shown in Figures 14-16, the rotational nut 1795 is rotationally fixed relative to the handle 124, but axially moveable relative to the handle 124. As described in further detail below, the rotational coupling between the shaft stop 1794 and the rotational nut 1795 defines the range of motion for rotation of the shaft 126 relative to the handle 124.

As shown in Figure 19, the shaft stop 1794 includes a through-bore 1994A extending between a proximal end 1994B and a distal end 1994C of the shaft stop 1794, a shaft coupling mechanism 1994D, and an external thread 1994E. The through-bore 1994A is configured to receive a proximal portion of the shaft 126 such that the shaft stop 1794 surrounds the proximal portion of the shaft 126, as shown in Figures 17 and 20.

The shaft coupling mechanism 1994D is configured to fixedly couple the shaft stop 1794 to the proximal portion of the shaft 126. In Figure 19, for example, the shaft coupling mechanism 1994D includes a plurality of tabs that are configured to engage and couple to a plurality of apertures 1896 on the proximal portion of the shaft 126 when the proximal portion of the shaft 126 is received in the through-bore 1994A of the shaft stop 1794. Figure 18 shows the apertures 1896 on the proximal portion of the shaft 126, and Figure 20 shows the tabs of the shaft coupling mechanism 1994D engaging the apertures 1896 to rotationally and axially fix the shaft stop 1794 relative to the shaft 126.

The shaft stop 1794 is rotationally coupled to the rotational nut 1795. For example, as shown in Figure 21, the rotational nut 1795 can include an internal thread 2195A that can threadably engage the external thread 1994E of the shaft stop 1794. The range of motion between the shaft 126 and the handle 124 corresponds to a range of motion between the shaft stop 1794 and the rotational nut 1795, which is defined the external thread 1994E of the shaft stop 1794 and the internal thread 2195A of the rotational nut 1795. For instance, the range of motion can be defined by a length and/or a number of turns of the external thread 1994E and/or the internal thread 2195A.

Referring to Figure 21, the rotational nut 1795 can include one or more protrusions 2195B that are configured to assist in preventing rotation of the rotational nut 1795 relative to the handle 124 and allow axial movement of the rotational nut 1795A relative to the handle 124. For example, as shown in Figures 15-16, the handle 124 can include one or more longitudinal slots 1597 that are each configured to receive a respective one of the protrusions 2195B of the rotational nut 1795A. The longitudinal slots 1597 extend in a longitudinal direction (e.g., parallel to the longitudinal axis 350 shown in Figure 3) with a length that is greater than or equal to a length by which the shaft 126 can axially move relative to the handle 124. In this arrangement, an engagement between the protrusions 2195B and the longitudinal slots 1597 prevents rotation of the rotational nut 1795 relative to the handle 124 and allows axial movement of the rotational nut 1795 relative to the handle 124.

In Figures 14-21, the handle 124 includes two longitudinal slots 1597 on an upper internal surface 1598 and two longitudinal slots 1597 on a lower internal surface 1698. As shown in Figure 21, the rotational nut 1795 includes a corresponding two protrusions 2195B on an upper external surface that are configured to mate with the two longitudinal slots 1597 on the upper internal surface 1598, and a corresponding two protrusions 2195B on a lower external surface that are configured to mate with the two longitudinal slots 1597 on the lower internal surface 1698. Figures 14-16 show the protrusions 2195B in the longitudinal slots 1597 of the handle 124. In this arrangement, the rotational nut 1795 (i) moves axially with the shaft 126 (e.g., due to the coupling between the rotational nut 1795 with the shaft stop 1794, which is fixedly coupled to the shaft 126), (ii) is prevented from rotation relative to the handle 124 due to the engagement between the protrusions 2195B and the longitudinal slots 1597, and (iii) restricts rotation of the shaft 126 and the handle 124 to the predefined range of rotational movement by the threaded engagement with the shaft stop 1794 while being rotationally fixed relative to the handle 124.

In Figures 14-21, the rotational nut 1795 is C-shaped (e.g., an inner surface of a bore of the rotational nut 1795 does not extend around an entire circumference of the bore of the rotational nut 1795). For instance, the inner surface of the rotational nut 1795 and an outer surface of the rotational nut 1795 can extend as an arc around a center axis through the bore of the rotational nut 1795 by approximately 270 degrees to approximately 350 degrees. In this arrangement, the rotational nut 1795 can have opposing terminal ends that define an opening through a lateral wall of the rotational nut 1795 (e.g., between the bore of the rotational nut 1795 and an exterior of the bore). This can, for example, assist with assembly. For instance, the C-shape of the rotational nut 1795 can provide for the rotational nut 1795 flexing and/or bending to increase a size of the opening between the terminal ends so that the shaft stop 1794 can be inserted through the opening and into the bore of the rotational nut 1795. After inserting the shaft stop 1794 through the opening and into the bore of the rotational nut 1795, the rotational nut 1795 can elastically reduce in size again around the shaft stop 1794. In some examples, the shaft stop 1794 can be inserted through the opening in the rotational nut 1795 while the printed circuit board 132 is in the through-bore 1994A of the shaft stop 1794.

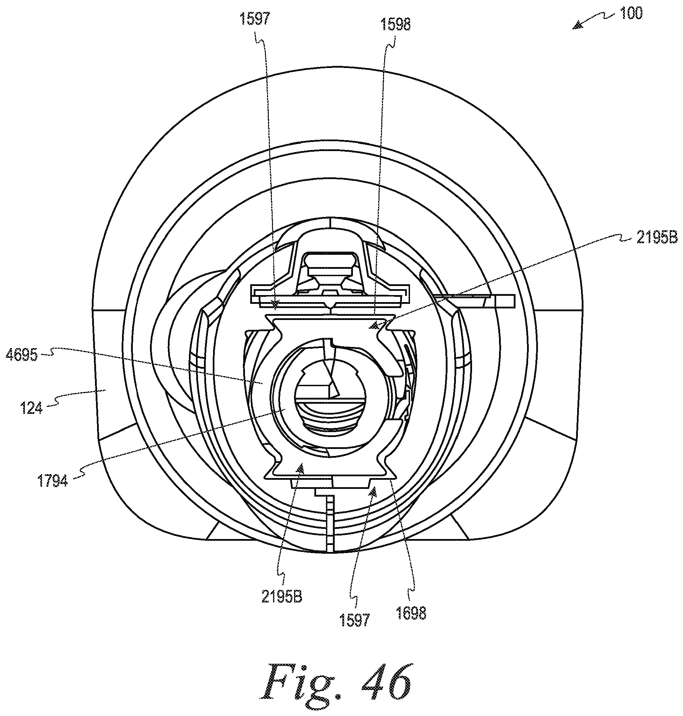

Figure 46 depicts a cross-sectional view of the electrosurgical tool 112 through the handle 124, the shaft stop 1794, and the rotational nut 1795, according to another example. The example shown in Figure 46 differs from the example shown and described above with respect to Figures 14-21 in that the protrusions 2195B and the longitudinal slots 1597 for non-rotational engagement between the handle 124 and the rotational nut 1795 have a different configuration than similar features shown in Figures 17-21. For instance, as shown in Figure 46, each of protrusions 2195B of the rotational nut 1795 can have a dovetail shape that is received in the respective longitudinal slot 1597 of the handle 124 having corresponding dovetail shapes. In this arrangement, an engagement between the protrusions 2195B and the longitudinal slots 1597 prevents rotation of the rotational nut 1795 relative to the handle 124 and allows axial movement of the rotational nut 1795 relative to the handle 124 as described above with respect to the rotational nut 1795.

In Figure 46, the handle 124 includes one longitudinal slot 1597 on the upper internal surface 1598 and one longitudinal slot 1597 on the lower internal surface 1698. As shown in Figure 46, the rotational nut 1795 includes a corresponding dovetail-shaped protrusion 2195B on an upper external surface that is configured to mate with the dovetail-shaped longitudinal slot 1597 on the upper internal surface 1598, and a corresponding dovetail-shaped protrusions 2195B on a lower external surface that is configured to mate with the dovetail-shaped longitudinal slot 1597 on the lower internal surface 1698. In this arrangement, the rotational nut 1795 (i) moves axially with the shaft 126 (e.g., due to the coupling between the rotational nut 1795 with the shaft stop 1794, which is fixedly coupled to the shaft 126), (ii) is prevented from rotation relative to the handle 124 due to the engagement between the protrusions 2195B and the longitudinal slots 1597, and (iii) restricts rotation of the shaft 126 and the handle 124 to the predefined range of rotational movement by the threaded engagement with the shaft stop 1794 while being rotationally fixed relative to the handle 124.

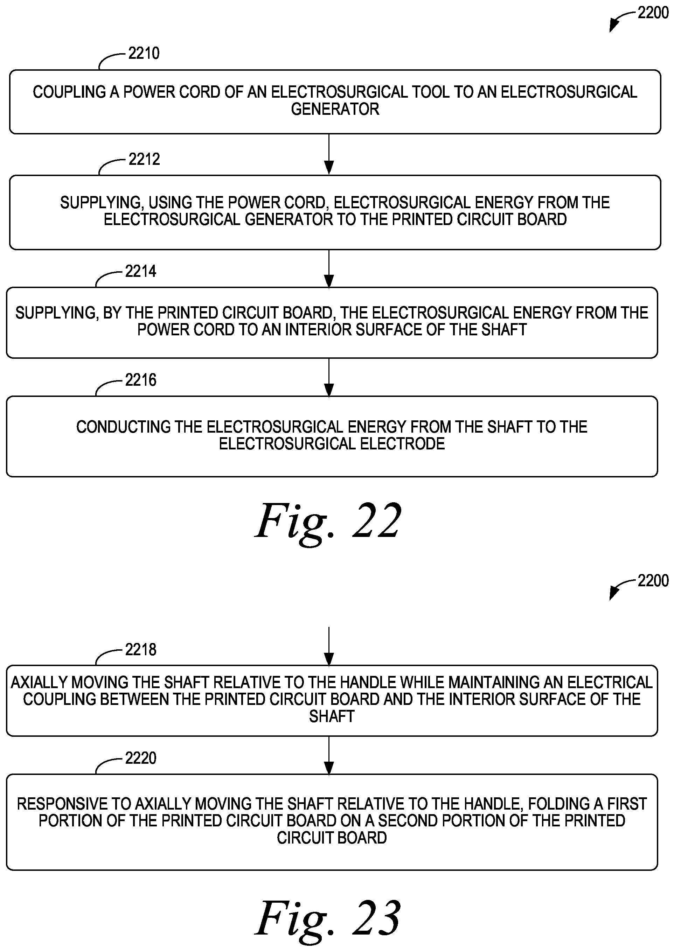

Referring now to Figure 22, a process 2200 of operating an electrosurgical tool is shown according to an example. As shown in Figure 22, at block 2210, the process 2200 includes coupling a power cord of an electrosurgical tool to an electrosurgical generator. The electrosurgical tool includes a handle defining an interior cavity, and a shaft extending distally from the interior cavity of the handle. The shaft defines an interior bore. The electrosurgical tool also includes a printed circuit board in the interior bore of the shaft. The printed circuit board is electrically coupled to the power cord. The shaft is movable relative to the handle and the printed circuit board. The electrosurgical tool further includes an electrosurgical electrode extending distally from a distal end of the shaft.

At block 2212, the process 2200 includes supplying, using the power cord, electrosurgical energy from the electrosurgical generator to the printed circuit board. At block 2214, the process 2200 includes supplying, by the printed circuit board, the electrosurgical energy from the power cord to an interior surface of the shaft. At block 2216, the process 2200 includes conducting the electrosurgical energy from the shaft to the electrosurgical electrode.

Figures 23-33 depict additional aspects of the process 2200 according to further examples. As shown in Figure 23, the process 2200 can also include axially moving the shaft relative to the handle while maintaining an electrical coupling between the printed circuit board and the interior surface of the shaft at block 2218 and, responsive to axially moving the shaft relative to the handle at block 2218, folding a first portion of the printed circuit board on a second portion of the printed circuit board at block 2220.

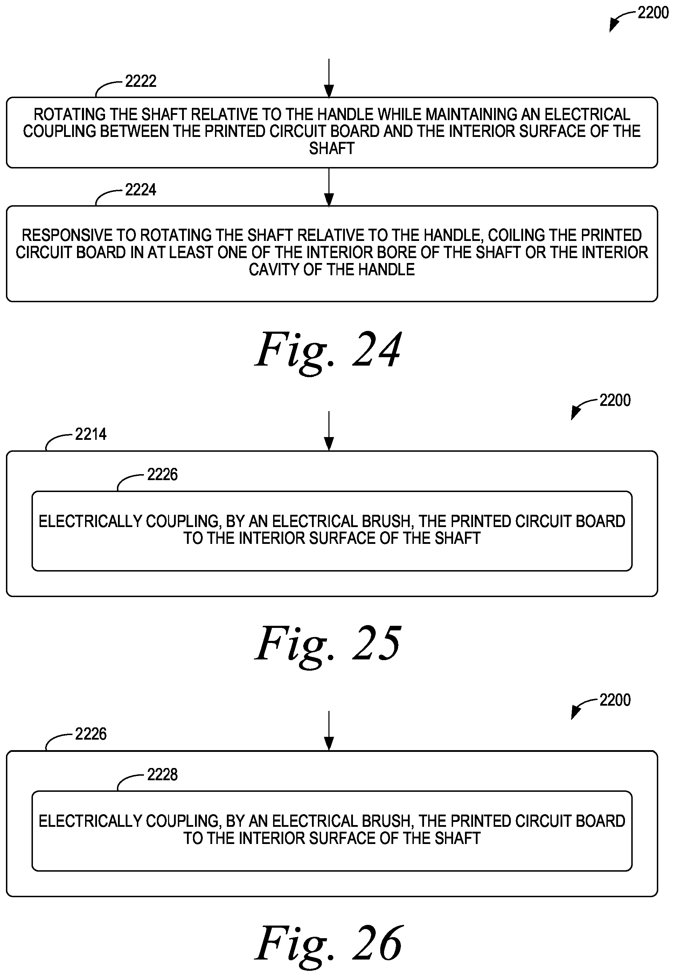

As shown in Figure 24, the process 2200 can include rotating the shaft relative to the handle while maintaining an electrical coupling between the printed circuit board and the interior surface of the shaft at block 2222 and, responsive to rotating the shaft relative to the handle at block 2222, coiling the printed circuit board in at least one of the interior bore of the shaft or the interior cavity of the handle at block 2224.

As shown in Figure 25, supplying, by the printed circuit board, the electrosurgical energy from the power cord to an interior surface of the shaft at block 2214 can include electrically coupling, by an electrical brush, the printed circuit board to the interior surface of the shaft at block 2226.

As shown in Figure 26, electrically coupling, by an electrical brush, the printed circuit board to the interior surface of the shaft at block 2226 can include pressing the printed circuit board between the electrical brush and a heatsink in the interior bore of the shaft at block 2228.

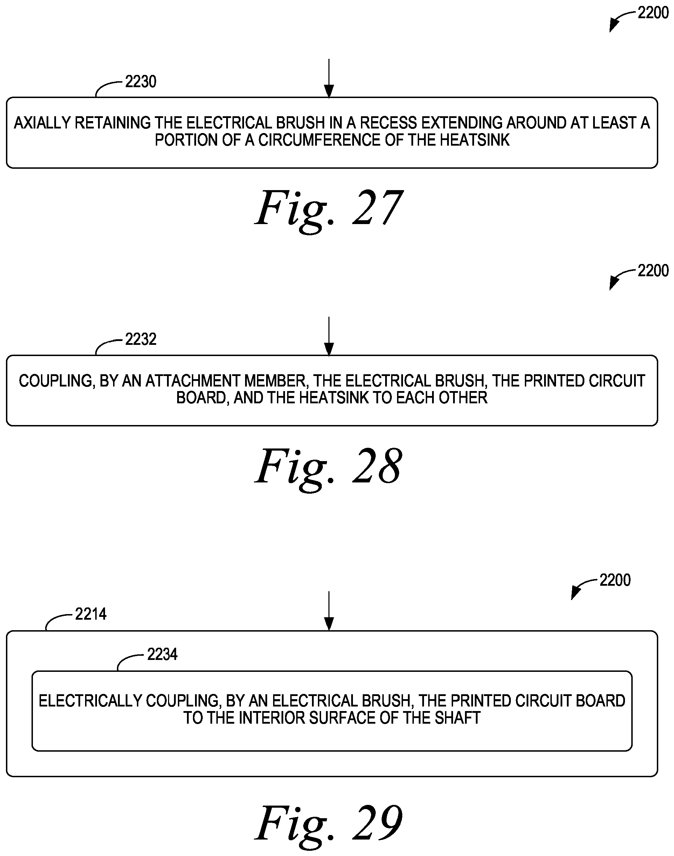

As shown in Figure 27, the process 2200 can include also include axially retaining the electrical brush in a recess extending around at least a portion of a circumference of the heatsink at block 2230.

As shown in Figure 28, the process 2200 can also include coupling, by an attachment member, the electrical brush, the printed circuit board, and the heatsink to each other at block 2232.

As shown in Figure 29, supplying, by the printed circuit board, the electrosurgical energy from the power cord to an interior surface of the shaft at block 2214 can include forcing, by a biasing member, a conductive contact of the printed circuit board into contact with the interior surface of the shaft at block 2234.

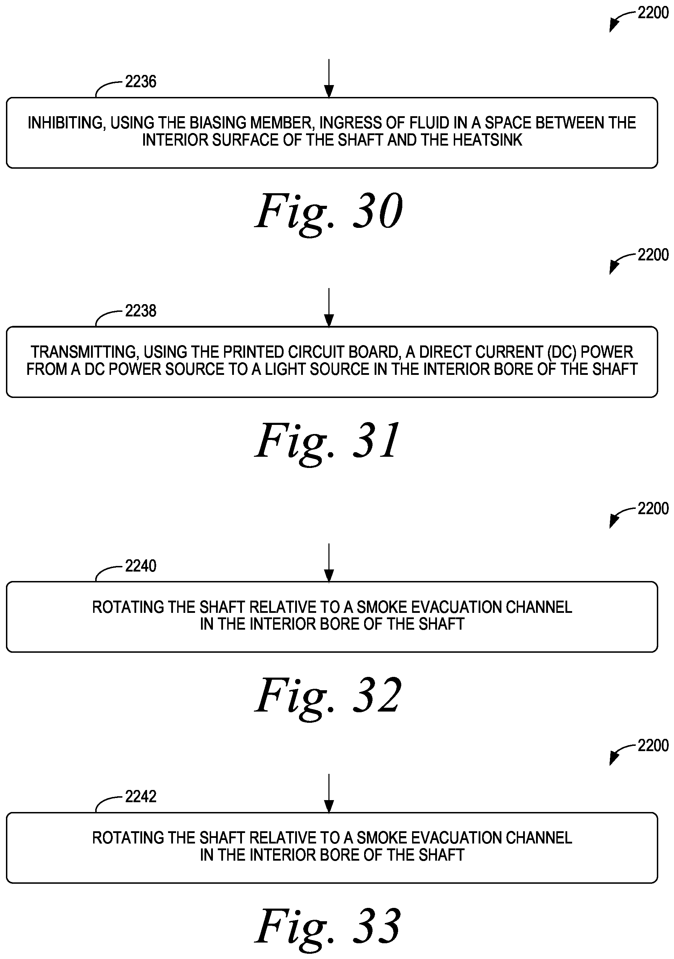

As shown in Figure 30, the process 2200 can also include inhibiting, using the biasing member, ingress of fluid in a space between the interior surface of the shaft and the heatsink at block 2236.

As shown in Figure 31, the process 2200 can also include transmitting, using the printed circuit board, a direct current (DC) power from a DC power source to a light source in the interior bore of the shaft at block 2238.

As shown in Figure 32, the process 2200 can also include rotating the shaft relative to a smoke evacuation channel in the interior bore of the shaft at block 2240.

As shown in Figure 33, the process 2200 can also include restricting, by a stop assembly of the electrosurgical tool, rotation of the shaft relative to the handle to a predefined range of rotational movement at block 2242.

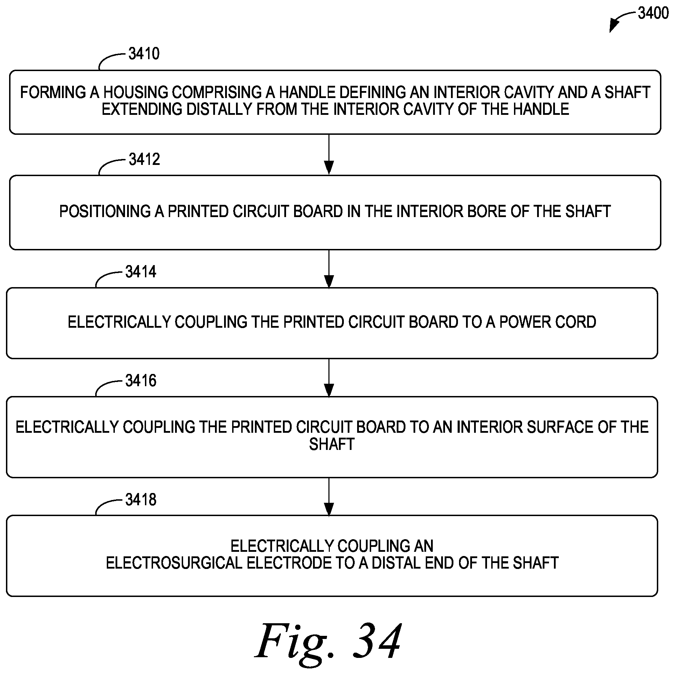

Referring now to Figure 34, a process 3400 of making an electrosurgical tool is shown according to an example. As shown in Figure 34, at block 3410, the process 3400 includes forming a housing comprising a handle defining an interior cavity and a shaft extending distally from the interior cavity of the handle. The shaft defines an interior bore. At block 3412, the process 3400 includes positioning a printed circuit board in the interior bore of the shaft. At block 3414, the process 3400 includes electrically coupling the printed circuit board to a power cord. The power cord is configured to couple to and receive electrosurgical energy from an electrosurgical generator. The shaft is movable relative to the handle and the printed circuit board. At block 3416, the process 3400 includes electrically coupling the printed circuit board to an interior surface of the shaft. At block 3418, the process 3400 includes electrically coupling an electrosurgical electrode to a distal end of the shaft. The shaft is configured to conduct the electrosurgical energy to the electrosurgical electrode.

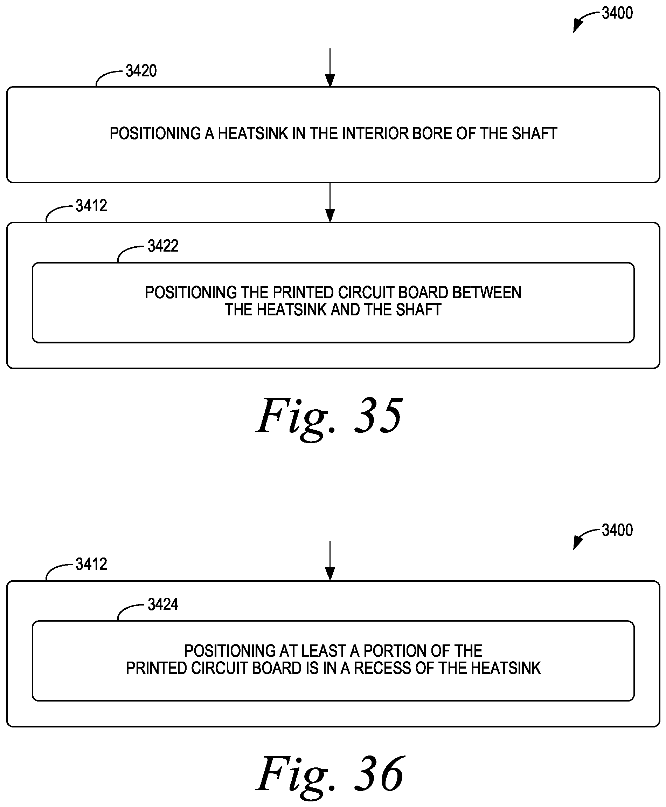

Figures 35-45 depict additional aspects of the process 3400 according to further examples. As shown in Figure 35, the process 3400 can include positioning a heatsink in the interior bore of the shaft at block 3420. Also, in Figure 35, positioning the printed circuit board in the interior bore of the shaft at block 3412 can include positioning the printed circuit board between the heatsink and the shaft at block 3422.

As shown in Figure 36, positioning the printed circuit board in the interior bore of the shaft at block 3412 can include positioning at least a portion of the printed circuit board is in a recess of the heatsink at block 3424. The recess can extend around at least a portion of a circumference of the heatsink.

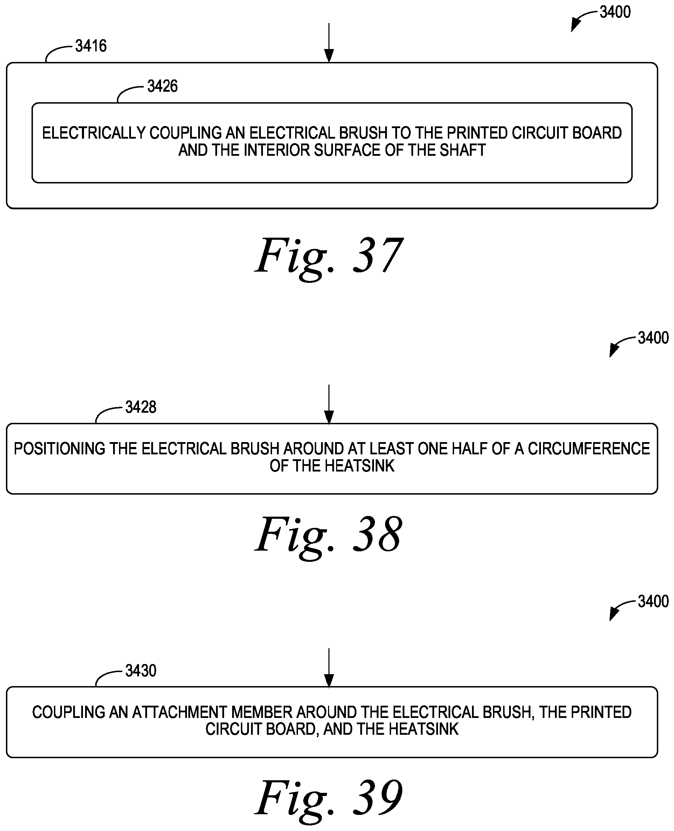

As shown in Figure 37, electrically coupling the printed circuit board to an interior surface of the shaft at block 3416 can include electrically coupling an electrical brush to the printed circuit board and the interior surface of the shaft at block 3426.

As shown in Figure 38, the process 3400 can also include positioning the electrical brush around at least one half of a circumference of the heatsink at block 3428.

As shown in Figure 39, the process 3400 can also include coupling an attachment member around the electrical brush, the printed circuit board, and the heatsink at block 3430.

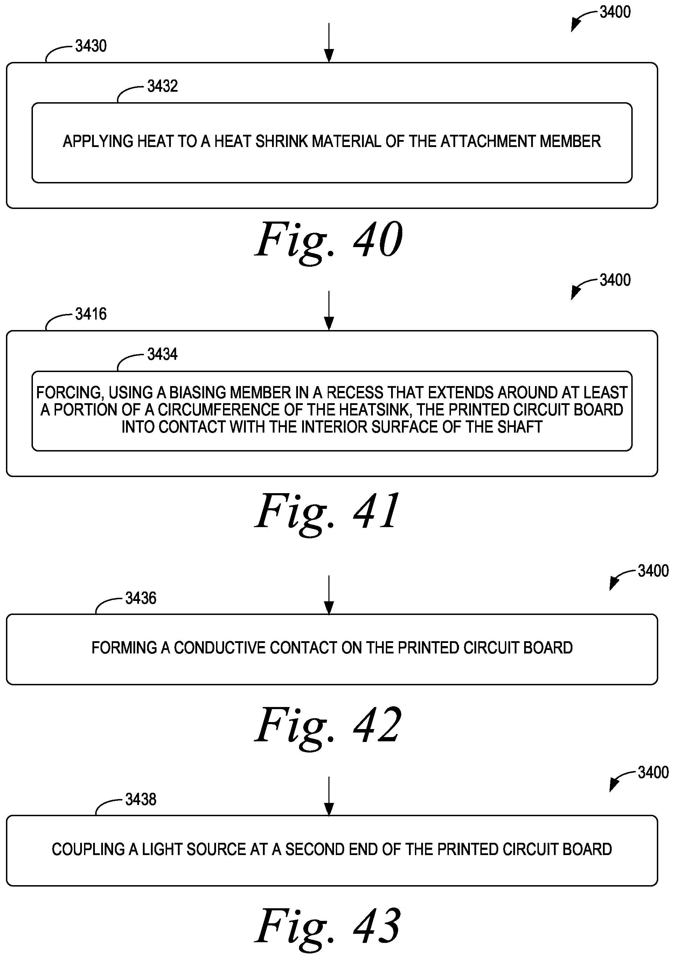

As shown in Figure 40, coupling the attachment member around the electrical brush, the printed circuit board, and the heatsink at block 3430 can include applying heat to a heat shrink material of the attachment member at block 3432.

As shown in Figure 41, electrically coupling the printed circuit board to an interior surface of the shaft at block 3416 can include forcing, using a biasing member in a recess that extends around at least a portion of a circumference of the heatsink, the printed circuit board into contact with the interior surface of the shaft at block 3434.

As shown in Figure 42, the process 3400 can also include forming a conductive contact on the printed circuit board at block 3436. A width of the printed circuit board at the conductive contact can be greater than a width of the printed circuit board at a first section of the printed circuit board that is proximal of the conductive contact and a second section of the printed circuit board that is distal of the conductive contact.

As shown in Figure 43, the process 3400 can also include coupling a light source at a second end of the printed circuit board at block 3438. The printed circuit board can have a first end and the second end, the printed circuit board can be elongated in an axial dimension extending between the first end and the second end, and the axial dimension can be parallel to a longitudinal axis of the shaft.

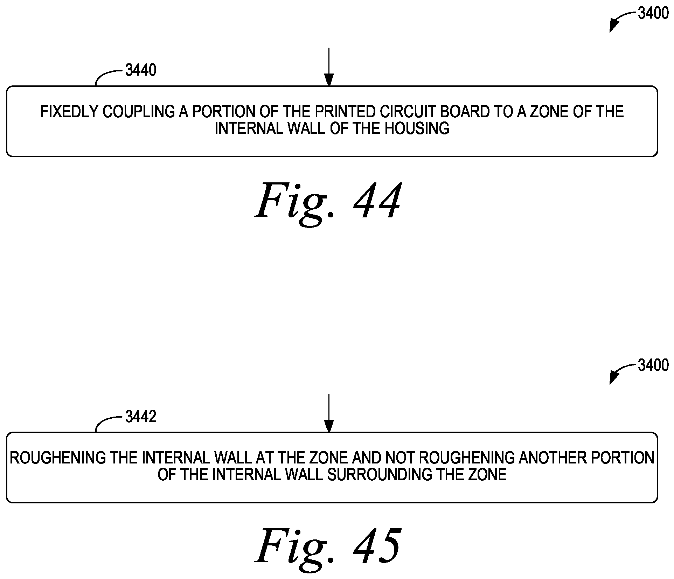

As shown in Figure 44, the process 3400 can also include fixedly coupling a portion of the printed circuit board to a zone of the internal wall of the housing at block 3440

As shown in Figure 45, the process 3400 can also include roughening the internal wall at the zone and not roughening another portion of the internal wall surrounding the zone at block 3442.

## Claims

1. An electrosurgical tool (112), comprising:
a handle (124) defining an interior cavity (252);
a power cord (122) configured to couple to and receive electrosurgical energy from an electrosurgical generator (110);
a shaft (126) extending distally from the interior cavity (252) of the handle (124), wherein the shaft defines an interior bore (254);
a printed circuit board (132) in the interior bore (254) of the shaft (126), wherein the printed circuit board is electrically coupled to the power cord (122), wherein the printed circuit board (132) is configured to conduct the electrosurgical energy from the power cord (122) to an interior surface (526A, 626A) of the shaft (126), wherein the shaft is at least one of: (i) rotatable relative to the handle (124) and the printed circuit board (132) or (ii) telescopically movable relative to the handle (124) and the printed circuit board (132); and
an electrosurgical electrode (128) extending distally from a distal end (126B) of the shaft (126), wherein the shaft is configured to conduct the electrosurgical energy to the electrosurgical electrode.

2. The electrosurgical tool (112) of claims 1, further comprising a heatsink (458) in the interior bore (254) of the shaft (126), and
wherein the printed circuit board (132) is between the heatsink (458) and the shaft (126).

3. The electrosurgical tool (112) of claim 2, wherein the heatsink (458) comprises a recess (568, 668) extending around at least a portion of a circumference of the heatsink (458),
wherein at least a portion of the printed circuit board (132) is in the recess (568, 668) of the heatsink (458).

4. The electrosurgical tool (112) of claim 3, further comprising an electrical brush (562) that electrically couples the printed circuit board (132) to the interior surface (526A, 626A) of the shaft (126), optionally, wherein
i) the electrical brush (562) extends around at least one half of a circumference of the heatsink (458), and/or
ii) the electrosurgical tool (112) further comprises an attachment member (566) that couples the electrical brush (562), the printed circuit board (132), and the heatsink (458) to each other, preferably, wherein the attachment member (566) is a band formed of a heat shrink material.

5. The electrosurgical tool (112) of claim 4, wherein the electrical brush (562) comprises a distal end (570), a proximal end (572), and a center portion (574) between the distal end and the proximal end,
wherein the center portion (574) has a shape that corresponds to a shape of a conductive contact of printed circuit board (132) in the recess (568, 668) of the heatsink (458), and
wherein the distal end (570) of the electrical brush (562) and the proximal end (572) of the electrical brush extend radially outward from the center portion (574) to directly contact the interior surface (526A, 626A) of the shaft (126).

6. The electrosurgical tool (112) of claim 2, further comprising a biasing member (676) in a recess (568, 668) that extends around at least a portion of a circumference of the heatsink (458),
wherein the biasing member (676) is between the heatsink (458) and the printed circuit board (132), and
wherein the biasing member (676) forces the printed circuit board (132) into contact with the interior surface (526A, 626A) of the shaft (126), optionally, wherein
i) the biasing member (676) is an O-ring, and/or
ii) the biasing member (676) extends around an entire circumference of the heatsink (458) and provides a gasket that inhibits ingress of fluid in a space between the interior surface (526A, 626A) of the shaft (126) and the heatsink (458).

7. The electrosurgical tool (112) of any one of claims 1-6, wherein the printed circuit board (132) has a first end (132A) and a second end (132B),
wherein the printed circuit board (132) is elongated in an axial dimension extending between the first end (132A) and the second end (132B), and
wherein the axial dimension is parallel to a longitudinal axis (350) of the shaft (126).

8. The electrosurgical tool (112) of claim 7, wherein the printed circuit board (132) comprises a conductive contact (564) for electrically coupling the printed circuit board to the interior surface (526A, 626A) of the shaft (126), and
wherein the conductive contact (564) is between the first end (132A) and the second end (132B) of the printed circuit board (132), optionally, wherein
i) a width of the printed circuit board (132) at the conductive contact (564) is greater than a width of the printed circuit board at a first section of the printed circuit board that is proximal of the conductive contact (564) and a second section of the printed circuit board that is distal of the conductive contact (564), preferably, wherein the conductive contact (564) has a triangular shape, and/or
ii) the printed circuit board (132) comprises a light source (140) at the second end (132B) of the printed circuit board, and/or
iii) the printed circuit board (132) comprises a plurality of switches (138) that are actuatable by a plurality of buttons (330A, 330B) on the handle (124) to control the electrosurgical energy supplied to the electrosurgical electrode (128).

9. The electrosurgical tool (112) of any one of claims 1-8, wherein the printed circuit board (132) is a flexible printed circuit board.

10. The electrosurgical tool (112) of any one of claims 1-9, wherein a portion of the printed circuit board (132) is fixedly coupled to an internal wall (1524) of the handle (124), and wherein, optionally, the portion of the printed circuit board (132) is fixedly coupled to a zone (1501) of the internal wall (1524) of the handle (124), and wherein the zone (1501) is elongated along a longitudinal axis of the electrosurgical tool (112).

11. The electrosurgical tool (112) of any one of claims 1-9, wherein a portion of the printed circuit board (132) is fixedly coupled to an internal wall (1524) of the handle (124), and wherein, optionally, the portion of the printed circuit board (132) is fixedly coupled to a zone (1501) of the internal wall (1524) of the handle (124), and wherein the zone (1501) has a surface roughness that is greater than a surface roughness of another portion of the internal wall (1524) surrounding the zone (1501).

12. The electrosurgical tool (112) of any one of claims 1-11, further comprising a stop assembly (1793) that is configured to limit a range of rotational movement between the shaft (126) and the handle (124) to a predefined range of rotational movement, wherein, preferably, the predefined range of rotational movement is approximately 400 degrees.

13. The electrosurgical tool (112) of claim 12, wherein the stop assembly (1793) comprises:
a shaft stop (1794) fixedly coupled to a proximal portion of shaft (126); and
a rotational nut (1795) that is (i) rotatably coupled to the shaft stop (1794), (ii) rotationally fixed relative to the handle (124), and (iii) axially moveable relative to the handle (124)

14. The electrosurgical tool of claim 13, wherein
i) the shaft stop (1794) comprises an external thread (1994E), and the rotational nut (1795) comprises an internal thread (2195A) that threadably engages the external thread (1994E) of the shaft stop (1794), and
wherein the predefined range of motion between the shaft (126) and the handle (124) corresponds to a range of motion between the shaft stop (1794) and the rotational nut (1795), which is defined the external thread (1994E) of the shaft stop and the internal thread (2195A) of the rotational nut (1795), and/or
ii) the shaft stop (1794) comprises a plurality of tabs configured to engage and couple to a plurality of apertures (1896) on the proximal portion of the shaft (126) when the proximal portion of the shaft is received in a through-bore of the shaft stop (1794).

15. The electrosurgical tool (112) of claim 13 or 14, wherein the rotational nut (1795) comprises a plurality of protrusions (2195B),
wherein the handle (124) comprises a plurality of longitudinal slots (1597) that are each configured to receive a respective one of the protrusions (2195B) of the rotational nut (1795), and
wherein an engagement between the plurality of protrusions (2195B) and the plurality of longitudinal slots (1597) prevents rotation of the rotational nut (1795) relative to the handle (124) and allows axial movement of the rotational nut relative to the handle (124),,
wherein, preferably, each protrusion (2195B) has a dovetail shape and each longitudinal slot (1597) has a dovetail shape.

## Patentansprüche

1. Elektrochirurgisches Werkzeug (112), umfassend:
einen Handgriff (124), der einen Innenhohlraum (252) definiert;
ein Stromkabel (122), das dazu eingerichtet ist, an einen elektrochirurgischen Generator (110) zu koppeln und von diesem elektrochirurgische Energie zu empfangen;
einen Schaft (126), der sich distal von dem Innenhohlraum (252) des Handgriffs (124) erstreckt, wobei der Schaft eine Innenbohrung (254) definiert;
eine Leiterplatte (132) in der Innenbohrung (254) des Schafts (126), wobei die Leiterplatte elektrisch mit dem Stromkabel (122) gekoppelt ist,
wobei die Leiterplatte (132) dazu eingerichtet ist, die elektrochirurgische Energie vom Stromkabel (122) zu einer Innenfläche (526A, 626A) des Schafts (126) zu leiten, wobei der Schaft mindestens eines ist von: (i) drehbar relativ zu dem Handgriff (124) und der Leiterplatte (132) oder (ii) teleskopisch bewegbar relativ zu dem Handgriff (124) und der Leiterplatte (132); und
eine elektrochirurgische Elektrode (128), die sich distal von einem distalen Ende (126B) des Schafts (126) erstreckt, wobei der Schaft dazu eingerichtet ist, die elektrochirurgische Energie zur elektrochirurgischen Elektrode zu leiten.

2. Elektrochirurgisches Werkzeug (112) nach Anspruch 1, ferner umfassend einen Kühlkörper (458) in der Innenbohrung (254) des Schafts (126), und
wobei die Leiterplatte (132) sich zwischen dem Kühlkörper (458) und dem Schaft (126) befindet.

3. Elektrochirurgisches Werkzeug (112) nach Anspruch 2, wobei der Kühlkörper (458) eine Aussparung (568, 668) umfasst, die sich um mindestens einen Abschnitt eines Umfangs des Kühlkörpers (458) erstreckt,
wobei sich mindestens ein Abschnitt der Leiterplatte (132) in der Aussparung (568, 668) des Kühlkörpers (458) befindet.

4. Elektrochirurgisches Werkzeug (112) nach Anspruch 3, ferner umfassend eine elektrische Bürste (562), welche die Leiterplatte (132) elektrisch mit der Innenfläche (526A, 626A) des Schafts (126) koppelt, wobei, als Option,
i) die elektrische Bürste (562) sich um mindestens eine Hälfte eines Umfangs des Kühlkörpers (458) erstreckt, und/oder
ii) das elektrochirurgische Werkzeug (112) ferner ein Befestigungselement (566) umfasst, das die elektrische Bürste (562), die Leiterplatte (132) und den Kühlkörper (458) miteinander koppelt, wobei vorzugsweise das Befestigungselement (566) ein Band ist, das aus einem Wärmeschrumpfmaterial gebildet ist.

5. Elektrochirurgisches Werkzeug (112) nach Anspruch 4, wobei die elektrische Bürste (562) ein distales Ende (570), ein proximales Ende (572) und einen Mittelabschnitt (574) zwischen dem distalen Ende und dem proximalen Ende umfasst,
wobei der Mittelabschnitt (574) eine Form aufweist, die einer Form eines leitenden Kontakts der Leiterplatte (132) in der Aussparung (568, 668) des Kühlkörpers (458) entspricht, und
wobei sich das distale Ende (570) der elektrischen Bürste (562) und das proximale Ende (572) der elektrischen Bürste radial von dem Mittelabschnitt (574) nach außen erstrecken, um die Innenfläche (526A, 626A) des Schafts (126) direkt zu kontaktieren.

6. Elektrochirurgisches Werkzeug (112) nach Anspruch 2, ferner umfassend ein Vorspannelement (676) in einer Aussparung (568, 668), die sich um mindestens einen Abschnitt eines Umfangs des Kühlkörpers (458) erstreckt,
wobei sich das Vorspannelement (676) zwischen dem Kühlkörper (458) und der Leiterplatte (132) befindet, und
wobei das Vorspannelement (676) die Leiterplatte (132) in Kontakt mit der Innenfläche (526A, 626A) des Schafts (126) zwingt, wobei, als Option,
i) das Vorspannelement (676) ein O-Ring ist, und/oder
ii) das Vorspannelement (676) sich um einen gesamten Umfang des Kühlkörpers (458) erstreckt und eine Dichtung bereitstellt, die ein Eindringen von Fluid in einen Raum zwischen der Innenfläche (526A, 626A) des Schafts (126) und dem Kühlkörper (458) verhindert.

7. Elektrochirurgisches Werkzeug (112) nach einem der Ansprüche 1 bis 6, wobei die Leiterplatte (132) ein erstes Ende (132A) und ein zweites Ende (132B) aufweist,
wobei die Leiterplatte (132) in einer axialen Abmessung länglich ist, die sich zwischen dem ersten Ende (132A) und dem zweiten Ende (132B) erstreckt, und
wobei die axiale Abmessung parallel zu einer Längsachse (350) des Schafts (126) verläuft.

8. Elektrochirurgisches Werkzeug (112) nach Anspruch 7, wobei die Leiterplatte (132) einen leitenden Kontakt (564) zum elektrischen Koppeln der Leiterplatte mit der Innenfläche (526A, 626A) des Schafts (126) umfasst, und
wobei sich der leitende Kontakt (564) zwischen dem ersten Ende (132A) und dem zweiten Ende (132B) der Leiterplatte (132) befindet, wobei, als Option,
i) eine Breite der Leiterplatte (132) an dem leitenden Kontakt (564) größer ist als eine Breite der Leiterplatte an einem ersten Abschnitt der Leiterplatte, der proximal zu dem leitenden Kontakt (564) liegt, und einem zweiten Abschnitt der Leiterplatte, der distal zu dem leitenden Kontakt (564) liegt, wobei vorzugsweise der leitende Kontakt (564) eine dreieckige Form aufweist, und/oder
ii) die Leiterplatte (132) eine Lichtquelle (140) an dem zweiten Ende (132B) der Leiterplatte umfasst, und/oder
iii) die Leiterplatte (132) eine Mehrzahl von Schaltern (138) umfasst, die durch eine Mehrzahl von Knöpfen (330A, 330B) am Handgriff (124) betätigbar sind, um die elektrochirurgische Energie zu steuern, die der elektrochirurgischen Elektrode (128) zugeführt wird.

9. Elektrochirurgisches Werkzeug (112) nach einem der Ansprüche 1 bis 8, wobei die Leiterplatte (132) eine flexible Leiterplatte ist.

10. Elektrochirurgisches Werkzeug (112) nach einem der Ansprüche 1 bis 9, wobei ein Abschnitt der Leiterplatte (132) fest mit einer Innenwand (1524) des Handgriffs (124) gekoppelt ist, und wobei, als Option, der Abschnitt der Leiterplatte (132) fest mit einer Zone (1501) der Innenwand (1524) des Handgriffs (124) gekoppelt ist, und wobei die Zone (1501) entlang einer Längsachse des elektrochirurgischen Werkzeugs (112) länglich ist.

11. Elektrochirurgisches Werkzeug (112) nach einem der Ansprüche 1 bis 9, wobei ein Abschnitt der Leiterplatte (132) fest mit einer Innenwand (1524) des Handgriffs (124) gekoppelt ist, und wobei, als Option, der Abschnitt der Leiterplatte (132) fest mit einer Zone (1501) der Innenwand (1524) des Handgriffs (124) gekoppelt ist, und wobei die Zone (1501) eine Oberflächenrauheit aufweist, die größer ist als eine Oberflächenrauheit eines anderen Abschnitts der Innenwand (1524), der die Zone (1501) umgibt.

12. Elektrochirurgisches Werkzeug (112) nach einem der Ansprüche 1 bis 11, ferner umfassend eine Anschlagsanordnung (1793), die dazu eingerichtet ist, einen Bereich der Drehbewegung zwischen dem Schaft (126) und dem Handgriff (124) auf einen vordefinierten Bereich der Drehbewegung zu begrenzen, wobei vorzugsweise der vordefinierte Bereich der Drehbewegung ungefähr 400 Grad beträgt.

13. Elektrochirurgisches Werkzeug (112) nach Anspruch 12, wobei die Anschlagsanordnung (1793) umfasst:
einen Schaftanschlag (1794), der fest mit einem proximalen Abschnitt des Schafts (126) gekoppelt ist; und
eine Drehmutter (1795), die (i) drehbar mit dem Schaftanschlag (1794) gekoppelt ist, (ii) relativ zu dem Handgriff (124) drehfest ist und (iii) relativ zu dem Handgriff (124) axial bewegbar ist.

14. Elektrochirurgisches Werkzeug nach Anspruch 13, wobei
i) der Schaftanschlag (1794) ein Außengewinde (1994E) umfasst und die Drehmutter (1795) ein Innengewinde (2195A) umfasst, das gewindemäßig in das Außengewinde (1994E) des Schaftanschlags (1794) eingreift, und
wobei der vordefinierte Bewegungsbereich zwischen dem Schaft (126) und dem Handgriff (124) einem Bewegungsbereich zwischen dem Schaftanschlag (1794) und der Drehmutter (1795) entspricht, der durch das Außengewinde (1994E) des Schaftanschlags und das Innengewinde (2195A) der Drehmutter (1795) definiert ist, und/oder
ii) der Schaftanschlag (1794) eine Mehrzahl von Nasen umfasst, die dazu eingerichtet sind, in eine Mehrzahl von Öffnungen (1896) am proximalen Abschnitt des Schafts (126) einzugreifen und daran zu koppeln, wenn der proximale Abschnitt des Schafts in einer Durchgangsbohrung des Schaftanschlags (1794) aufgenommen ist.

15. Elektrochirurgisches Werkzeug (112) nach Anspruch 13 oder 14, wobei die Drehmutter (1795) eine Mehrzahl von Vorsprüngen (2195B) umfasst,
wobei der Handgriff (124) eine Mehrzahl von Längsschlitzen (1597) umfasst, die jeweils dazu eingerichtet sind, einen entsprechenden der Vorsprünge (2195B) der Drehmutter (1795) aufzunehmen, und
wobei ein Eingriff zwischen der Mehrzahl von Vorsprüngen (2195B) und der Mehrzahl von Längsschlitzen (1597) eine Drehung der Drehmutter (1795) relativ zu dem Handgriff (124) verhindert und eine axiale Bewegung der Drehmutter relativ zu dem Handgriff (124) ermöglicht,
wobei vorzugsweise jeder Vorsprung (2195B) eine Schwalbenschwanzform aufweist und jeder Längsschlitz (1597) eine Schwalbenschwanzform aufweist.

## Revendications

1. Outil électrochirurgical (112) comprenant:
un manche (124) formant une cavité intérieure (252);
un cordon d'alimentation (122) conçu pour se connecter à un générateur électrochirurgical (110) qui l'alimente en énergie électrochirurgicale;
une tige (126) s'étendant distalement à partir de la cavité intérieure (252) du manche (124), la tige formant un alésage intérieur (254);
une carte de circuit imprimé (132) dans l'alésage intérieur (254) de la tige (126), la carte de circuit imprimé étant accouplée électriquement au cordon d'alimentation (122),
dans lequel la carte de circuit imprimé (132) étant conçue pour conduire l'énergie électrochirurgicale du cordon d'alimentation (122) à une face intérieure (526A, 626A) de la tige (126), la tige étant au moins: (i) rotative par rapport au manche (124) et à la carte de circuit imprimé (132) ou (ii) télescopique par rapport au manche (124) et à la carte de circuit imprimé (132); et
une électrode électrochirurgicale (128) s'étendant de manière distale à partir d'une extrémité distale (126B) de la tige (126), la tige étant conçue pour conduire l'énergie électrochirurgicale à l'électrode électrochirurgicale.

2. Outil électrochirurgical (112) selon la revendication 1, comprenant en outre un dissipateur thermique (458) dans l'alésage intérieur (254) de la tige (126), et
dans lequel la carte de circuit imprimé (132) est située entre le dissipateur thermique (458) et la tige (126).

3. Outil électrochirurgical (112) selon la revendication 2, dans lequel le dissipateur thermique (458) comprend un évidement (568, 668) s'étendant autour d'au moins une partie de la circonférence du dissipateur thermique (458),
dans lequel au moins une partie de la carte de circuit imprimé (132) se situe dans l'évidement (568, 668) du dissipateur thermique (458).

4. Outil électrochirurgical (112) selon la revendication 3, comprenant en outre une brosse électrique (562) qui couple électriquement la carte de circuit imprimé (132) à la face intérieure (526A, 626A) de la tige (126), éventuellement, dans lequel
i) la brosse électrique (562) s'étend sur au moins la moitié de la circonférence du dissipateur thermique (458), et/ou
ii) l'outil électrochirurgical (112) comprend en outre un élément de fixation (566) qui relie entre eux la brosse électrique (562), la carte de circuit imprimé (132) et le dissipateur thermique (458), de préférence, l'élément de fixation (566) étant une bande formée d'un matériau thermorétractable.

5. Outil électrochirurgical (112) selon la revendication 4, dans lequel la brosse électrique (562) comprend une extrémité distale (570), une extrémité proximale (572) et une partie centrale (574) entre l'extrémité distale et l'extrémité proximale,
dans lequel la partie centrale (574) a une forme qui correspond à la forme d'un contact conducteur de la carte de circuit imprimé (132) dans l'évidement (568, 668) du dissipateur thermique (458), et
dans lequel l'extrémité distale (570) de la brosse électrique (562) et l'extrémité proximale (572) de la brosse électrique s'étendent radialement vers l'extérieur à partir de la partie centrale (574) pour entrer directement en contact avec la face intérieure (526A, 626A) de la tige (126).

6. Outil électrochirurgical (112) selon la revendication 2, comprenant en outre un élément de sollicitation (676) dans un évidement (568, 668) qui s'étend autour d'au moins une partie d'une circonférence du dissipateur thermique (458),
dans lequel l'élément de sollicitation (676) est situé entre le dissipateur thermique (458) et la carte de circuit imprimé (132), et
dans lequel l'élément de sollicitation (676) contraint la carte de circuit imprimé (132) à entrer en contact avec la face intérieure (526A, 626A) de la tige (126), éventuellement, dans lequel
i) l'élément de sollicitation (676) est un joint torique, et/ou
ii) l'élément de sollicitation (676) s'étend sur toute la circonférence du dissipateur thermique (458) et forme un joint qui empêche que le liquide n'entre dans un espace entre la face intérieure (526A, 626A) de la tige (126) et le dissipateur thermique (458).

7. Outil électrochirurgical (112) selon l'une quelconque des revendications 1 à 6, dans lequel la carte de circuit imprimé (132) comporte une première extrémité (132A) et une deuxième extrémité (132B),
dans lequel la carte de circuit imprimé (132) est allongée dans une dimension axiale s'étendant entre la première extrémité (132A) et la deuxième extrémité (132B), et
dans lequel la dimension axiale est parallèle à un axe longitudinal (350) de la tige (126).

8. Outil électrochirurgical (112) selon la revendication 7, dans lequel la carte de circuit imprimé (132) comprend un contact conducteur (564) servant à coupler électriquement la carte de circuit imprimé à la face intérieure (526A, 626A) de la tige (126), et
dans lequel le contact conducteur (564) est situé entre la première extrémité (132A) et la deuxième extrémité (132B) de la carte de circuit imprimé (132), éventuellement, dans lequel
i) la largeur de la carte de circuit imprimé (132) au niveau du contact conducteur (564) est supérieure à la largeur de la carte de circuit imprimé au niveau d'une première section de la carte de circuit imprimé qui est proximale par rapport au contact conducteur (564) et d'une deuxième section de la carte de circuit imprimé qui est distale par rapport au contact conducteur (564), de préférence, le contact conducteur (564) se présente sous une forme triangulaire, et/ou
ii) la carte de circuit imprimé (132) comprend une source lumineuse (140) à la deuxième extrémité (132B) de la carte de circuit imprimé, et/ou
iii) la carte de circuit imprimé (132) comprend une pluralité de commutateurs (138) qui peuvent être actionnés par une pluralité de boutons (330A, 330B) situés sur le manche (124) afin de régler l'énergie électrochirurgicale fournie à l'électrode électrochirurgicale (128).

9. Outil électrochirurgical (112) selon l'une quelconque des revendications 1 à 8, dans lequel la carte de circuit imprimé (132) est une carte de circuit imprimé flexible.

10. Outil électrochirurgical (112) selon l'une quelconque des revendications 1 à 9, dans lequel une partie de la carte de circuit imprimé (132) est couplée à demeure à une paroi interne (1524) du manche (124), et dans lequel, éventuellement, la partie de la carte de circuit imprimé (132) est couplée à demeure à une zone (1501) de la paroi interne (1524) du manche (124), et dans lequel la zone (1501) est allongée le long d'un axe longitudinal de l'outil électrochirurgical (112).

11. Outil électrochirurgical (112) selon l'une quelconque des revendications 1 à 9, dans lequel une partie de la carte de circuit imprimé (132) est couplée à demeure à une paroi interne (1524) du manche (124), et, éventuellement, la partie de la carte de circuit imprimé (132) étant couplée à demeure à une zone (1501) de la paroi interne (1524) du manche (124), et la zone (1501) présentant une rugosité de surface supérieure à la rugosité de surface d'une autre partie de la paroi interne (1524) entourant la zone (1501).

12. Outil électrochirurgical (112) selon l'une quelconque des revendications 1 à 11, comprenant en outre un ensemble butée (1793) qui est conçu pour limiter une plage de mouvement de rotation entre la tige (126) et le manche (124) à une plage de mouvement de rotation prédéfinie, de préférence, la plage de mouvement de rotation prédéfinie étant d'environ 400 degrés.

13. Outil électrochirurgical (112) selon la revendication 12, dans lequel l'ensemble butée (1793) comprend:
une butée de tige (1794) couplée à demeure à une partie proximale de la tige (126); et
un écrou rotatif (1795) qui est (i) couplé en rotation à la butée de tige (1794), (ii) fixé en rotation par rapport au manche (124), et (iii) mobile axialement par rapport au manche (124)

14. Outil électrochirurgical (112) selon la revendication 13, dans lequel
i) la butée de tige (1794) comprend un filetage externe (1994E), et l'écrou rotatif (1795) comprend un filetage interne (2195A) qui se visse sur le filetage externe (1994E) de la butée de tige (1794), et
dans lequel la plage de mouvement prédéfinie entre la tige (126) et le manche (124) correspond à une plage de mouvement entre la butée de tige (1794) et l'écrou rotatif (1795), qui est définie par le filetage externe (1994E) de la butée de tige et le filetage interne (2195A) de l'écrou rotatif (1795), et/ou
ii) la butée de tige (1794) comprend une pluralité de languettes conçues pour s'introduire et s'accoupler à une pluralité d'ouvertures (1896) sur la partie proximale de la tige (126) lorsque la partie proximale de la tige est reçue dans un alésage traversant de la butée de tige (1794).

15. Outil électrochirurgical (112) selon la revendication 13 ou 14, dans lequel l'écrou rotatif (1795) comprend une pluralité de saillies (2195B),
dans lequel le manche (124) comprend une pluralité de fentes longitudinales (1597) qui sont chacune conçues pour recevoir une saillie respective parmi les saillies (2195B) de l'écrou rotatif (1795), et
dans lequel une mise en prise entre la pluralité de saillies (2195B) et la pluralité de fentes longitudinales (1597) empêche la rotation de l'écrou rotatif (1795) par rapport au manche (124) et permet le mouvement axial de l'écrou rotatif par rapport au manche (124),
dans lequel, de préférence, chaque saillie (2195B) a une forme en queue d'aronde et chaque fente longitudinale (1597) a une forme en queue d'aronde.
